# EUROPEAN PATENT APPLICATION

(11) **EP 3 770 252 A1**
(43) Date of publication of application: **27.01.2021**
(21) Application number: 20193805.7
(22) Date of filing: 26.01.2016
(51) Int. Cl.: C12N 5/0789, C07J 5/00, C07K 14/555, C12N 15/11, A61K 35/12, A61P 37/02, C12N 5/0783, C12N 5/10

(54) **CELLS WITH INCREASED IMMUNO-REGULATORY PROPERTIES AND METHODS FOR THEIR USE AND MANUFACTURE**

(30) Priority: 26.01.2015 US 201562107517 P; 06.02.2015 US 201562112653 P
(62) Divisional of application: 16743971.0
(71) Applicant: Fate Therapeutics, Inc., San Diego, California 92121 (US)
(72) Inventor: FIORINA, Paolo, Boston, MA 02115 (US)
(74) Representative: Jones Day

(57) **Abstract**

The present invention is directed to a population of modified hematopoietic stem cells (HSCs), comprising an exogenous copy of a polynucleotide encoding PD-L1. The present invention also relates to pharmaceutical compositions comprising the modified HSCs and to their use in methods of treating immunological disorders.

## Description

### CROSS-REFERENCE TO RELATED APPLICATIONS

This application claims the benefit under 35 U.S.C. § 119(e) of U.S. Provisional Application No. 62/107,517 filed January 26, 2015 and U.S. Provisional Application No. 62/112,653 filed on February 6, 2015 which are each herein incorporated by reference in their entireties.

### BACKGROUND

Uncontrolled immune activation can be lethal, and so the immune system is tightly regulated, in part by pathways responsive to inflammation that modify immune cell functions.

PD-L1, also known as B7-H1, is a transmembrane protein that belongs to the B7 family of T cell co-inhibitory molecules. The binding of PD-L1 to its receptor PD-1 dampens T cell activation, decreases proliferation and cytotoxicity, and induces apoptosis. The immuno-regulatory property of hematopoietic stem and progenitor cells (HSPC) is enhanced upon increased expression of PD-L1. PD-L1 has been described in cancer immunotherapy for its role in blocking T cell activation and proliferation. More specifically, PD-L1 is capable of preventing T cell activation through competition for costimulatory molecules on the T cell (e.g. B7-1 and/or B7-2) and through direct engagement of PD1 on the T cell. Therefore, PD-L1 is capable of regulating T-cell activation in a cell contact dependent fashion. Moreover, the therapeutic potential of HSPC-based immunotherapies appears to be limited by the inherently low expression levels of PD-L1.

While increased levels of PD-L1 on HSPCs have been observed after culturing ex *vivo,* prolonged culture periods can result in replicative stress, stochastic cellular defects, and chromosomal abnormalities.

IDO-1 (indoleamine 2,3-dioxygenase) is an enzyme which catalyzes the degradation of the essential amino acid tryptophan (TRP). The depletion of tryptophan in the microenvironment halts T-cell proliferation, induces TH1 cell apoptosis, and activates regulatory T cells. This method of immune suppression is also naturally used by many other immunosuppressive cells, and is also used by many tumors to escape immune-activation. Although IDO enzymes are intracellular and not secreted, the metabolic effects of IDO-1 initially provide local effect, as neighboring cells respond to the reduced access to TRP. However, as the microenvironment is depleted of TRP, cells in proximity, but not in contact with the IDO-1 expressing cell are affected. Thus, in an autoimmune situation IDO-1 prevents T cell activation and proliferation by depleting TRP from the inflammatory microenvironment, and activates regulatory T cell suppression of the immune response. Expression of IDO-1 is very low in hematopoietic stem or progenitor cells under normal conditions. Thus, modulation of IDO-1 levels in hematopoietic stem and progenitor cells provides an opportunity to affect the immuno-regulatory properties of those cells, and upon administration, the immunological properties of patients' cells.

Thus, what is needed in the art is a method for producing HSPCs having increased PD-L1 and/or IDO-1 expression without exposing the cells to the stress of *in vitro* processes and prolonged cell culture.

The invention addresses these limitations through the identification of a number of molecules or compounds which, in a short-term incubation, independently or in combination, pharmacologically up- regulate PD-L1 and/or IDO-1 expression on cells, including HSPCs.

### BRIEF SUMMARY OF THE INVENTION

The present disclosure provides compositions and methods to modulate the immune system through the immuno-regulatory properties of cells expressing increased levels of programmed death ligand 1 (PD-L1) and indoleamine 2,3-dioxygenase 1 (IDO-1). The present disclosure is directed to compositions and methods to increase the expression of PD-L1 and/or IDO-1 in a population of cells, the modulated cells expressing increased PD-L1 and/or IDO-1, and methods related to the immunosuppressive effects obtained by cells expressing increased PD-L1 and/or IDO-1.

A first object of the invention includes methods for modulating a population of cells comprising: incubating the population of cells in the presence of one or more exogenous agents capable of increasing PD-L1 and/or IDO-1 expression to obtain a population of cells having increased expression of PD-L1 and/or IDO-1.

In one aspect, the incubation is *in vitro* or *ex vivo.*

In one aspect, the incubation is between about 5 minutes to about 72 hours. In a further aspect, the incubation is between about 4 hours to about 48 hours.

In one aspect, the incubation is performed at a temperature of between about 4°C to about 37°C. In a further aspect, the incubation is performed at a temperature of about 37°C.

In one aspect the increase in PD-L1 and/or IDO-1 expression in the modulated cells is at least 3-fold. In one aspect, the increase in PD-L1 and/or IDO-1 is about 3-fold to about 80-fold compared to cells not incubated with the exogenous agent.

In one aspect, the exogenous agent(s) are selected from one or more polynucleotides, one or more polypeptides, one or more small molecules, and combinations thereof. In one aspect, the polypeptide is an interferon receptor agonist. In a further aspect, the interferon receptor agonist is selected from IFN-α, IFN-β, IFN-ε, IFN-κ, IFN-ω, IFN-γ, or a combination thereof. In a particular aspect, the population of cells is modulated with IFN-β and IFN-γ.

In yet another aspect, the polynucleotide is selected from poly(I:C), a polynucleotide encoding PD-L1 and/or a polynucleotide encoding IDO-1.

In one particular aspect, at least two, at least three, or more exogenous agents are administered. In a particular aspect, IFN-β, IFN-γ, and poly(I:C) are administered.

In one aspect, the polynucleotide is selected from poly(I:C), a polynucleotide encoding PD-L1 and/or a polynucleotide encoding IDO-1.

In one aspect, the small molecules comprise glucocorticoids, prostaglandin pathway agonists antineoplastics, dopamine receptor agonists, isometheptene mucate, dihydrostreptomycin sulfate, protriptyline, telenzepine, cyclobenzaprine, 4-aminosalicylic acid and combinations thereof. In one aspect, the prostaglandin pathway agonist is selected from PGE₂, dmPGE₂, 15(S)-15-methyl PGE₂, 20-ethyl PGE₂, 8-iso-16-cyclohexyl-tetranor PGE₂, 16,16-dimethyl PGE₂ ("dmPGE2"), p-(p-acetamidobenzamido) phenyl ester, 11-deoxy-16, 16-dimethyl PGE₂, 9-deoxy- 9-methylene-16, 16-dimethyl PGE₂, 9-deoxy-9-methylene PGE₂, 9-keto Fluprostenol, 5-trans PGE₂, 17-phenyl-omega-trinor PGE₂, PGE₂ serinol amide, PGE₂ methyl ester, 16-phenyl tetranor PGE₂, 15(S)-15-methyl PGE₂, 15(R)-15-methyl PGE₂, 8-iso-15-keto PGE₂, 8-iso PGE₂ isopropyl ester, 8-iso-16-cyclohexyl-tetranor PGE₂, 20-hydroxy PGE₂, 20-ethyl PGE₂, 11-deoxy PGE₁, nocloprost, sulprostone, butaprost, 15-keto PGE₂, and 19 (R) hydroxy PGE₂.

In one aspect, the glucocorticoid is selected from medrysone, alclometasone, alclometasone dipropionate, amcinonide, beclometasone, beclomethasone dipropionate, betamethasone, betamethasone benzoate, betamethasone valerate, budesonide, ciclesonide, clobetasol, clobetasol butyrate, clobetasol propionate, clobetasone, clocortolone, cloprednol, cortisol, cortisone, cortivazol, deflazacort, desonide, desoximetasone, desoxycortone, desoxymethasone, dexamethasone, diflorasone, diflorasone diacetate, diflucortolone, diflucortolone valerate, difluorocortolone, difluprednate, fluclorolone, fluclorolone acetonide, fludroxycortide, flumetasone, flumethasone, flumethasone pivalate, flunisolide, flunisolide hemihydrate, fluocinolone, fluocinolone acetonide, fluocinonide, fluocortin, fluocoritin butyl, fluocortolone, fluorocortisone, fluorometholone, fluperolone, fluprednidene, fluprednidene acetate, fluprednisolone, fluticasone, fluticasone propionate, formocortal, halcinonide, halometasone, hydrocortisone, hydrocortisone acetate, hydrocortisone aceponate, hydrocortisone buteprate, hydrocortisone butyrate, loteprednol, meprednisone, 6a-methylprednisolone, methylprednisolone, methylprednisolone acetate, methylprednisolone aceponate, mometasone, mometasone furoate, mometasone furoate monohydrate, paramethasone, prednicarbate, prednisolone, prednisone, prednylidene, rimexolone, tixocortol, triamcinolone, triamcinolone acetonide and ulobetasol, as well as combinations thereof. In a further aspect, the glucocorticoid is selected from betamethasone, clobetasol proprionate, flumethasone, flucinolone acetonide, medrysone, hydrocortisone, triamcinolone, alclometasone, and dexamethasone.

In one aspect, the antineoplastics are selected from gemcitabine, letrozole, and fludarabine and the dopamine receptor antagonist is fluphernazine.

In one aspect, the population of cells comprises hematopoietic cells.

In one aspect, the population of cells is isolated. In a particular aspect, the population of hematopoietic cells is derived from cord blood, peripheral blood, bone marrow, or induced pluripotent stem cells (iPSCs).

In another aspect, the population of hematopoietic cells is obtained from iPSCs.

In one aspect, the population comprises hematopoietic stem/progenitor cells (HSPCs).

In one aspect, the population comprises at least about 50%, at least about 60%, at least about 70%, at least about 80%, at least about 85%, at least about 90%, at least about 95%, at least about 98%, or at least about 99% HSPCs. In one aspect, the population comprises a substantially pure population of HSPCs.

In one aspect, the population of cells is enriched for CD34+ HPSCs prior to contact with the exogenous agent.

A second object of the invention includes a population of cells having increased PD-L1 and/or IDO-1 expression obtained by the methods described in the first aspect and aspects thereof.

A third object of the invention includes a method of treating an immunological disorder comprising: administering a therapeutically effective amount of the population of cells obtained by the methods described in any of the embodiments and/or aspects above to a patient in need thereof.

In one aspect, the population of cells comprises hematopoietic cells. In one aspect, the population of hematopoietic cells is derived from cord blood, peripheral blood, bone marrow, or iPSCs.

In yet a further aspect, the population of cells comprises HSPCs.

In a further aspect, the population of cells comprises at least about 50%, at least about 50%, at least about 60%, at least about 70%, at least about 80%, at least about 85%, at least about 90%, at least about 95%, at least about 98%, or at least about 99% HSPCs. In a particular aspect, the population of cells comprises a substantially pure population of HSPCs.

In yet a further aspect, the population of cells is enriched for CD34+ HPSCs prior to contact with the exogenous agent.

In one aspect, the population of cells is allogeneic to the patient. In a further aspect, the population of cells is HLA matched with the patient. In yet a further aspect, the population of cells comprises haplotyped enhanced-HSPCs. In another aspect, the population of cells is partially HLA matched or unmatched with the patient.

In one aspect, the therapeutically effective amount of the population of cells comprises about 2 x 10⁶ to about 2 x 10¹⁰ CD34+ hematopoietic cells.

In one aspect, the method comprises more than one administration of a therapeutically effective amount of cells. In one aspect, the frequency of administrations ranges from about every other week to about every six months. In a further aspect, the initial administration is a higher number of cells than a subsequent administration.

In one emebodiment, the immunological disorder is an autoimmune disorder selected from acute myocardial infarction, ischemic stroke, type 1 diabetes, diabetes mellitus, multiple sclerosis, acute disseminated encephalomyelitis, inflammatory demyelinating diseases, lupus, Crohn's disease, osteoarthritis, rheumatoid arthritis, psoriatic arthritis, ulcerative colitis, dermatitis, irritable bowel syndrome, vitiligo, Graves' disease, Hashimoto's disease, Addison's disease, polymyositis, dermatomyositis, myasthenia gravis, autoimmune hepatitis, Sjögren's syndrome, autoimmune gastritis, sclerosis, psoriasis, asthma, or Wegener's granulomatosis.

In a particular aspect, the immunological disorder is graft vs host disease or transplant rejection. In a further aspect, the transplant rejections arose from a bone marrow transplant, solid organ transplant, or cell therapy (e.g. any composition comprising isolated stem cells).

In one aspect, the patient has undergone at least one of high-dose, reduced-intensity, or nonmyeloablative conditioning. In one aspect, the patient has not undergone at least one of high-dose, reduced-intensity, or nonmyeloablative conditioning. In yet a further aspect, the patient has not undergone conditioning.

In one aspect, the patient is not a candidate for cellular transplant or has not received a transplant.

A fourth object of the invention includes methods of treating inflammation in a patient comprising: administering a therapeutically effective amount of the population of cells obtained by the methods described in any of the embodiments and/or aspects above to a patient in need thereof.

In one aspect, the population of cells comprises hematopoietic cells.

In one aspect, the population of hematopoietic cells is derived from cord blood, peripheral blood, bone marrow, or iPSCs. In a particular aspect, the population comprises HSPCs.

In one aspect, the population comprises at least about 50% HPSCs, at least about 50%, at least about 60%, at least about 70%, at least about 80%, at least about 85%, at least about 90%, at least about 95%, at least about 98%, or at least about 99% HSPCs. In a further aspect, the population comprises a substantially pure population of HSPCs.

In one aspect, the population is enriched for CD34+ HPSCs prior to contact with the exogenous agent.

In a further aspect, the population of cells is allogeneic to the patient.

In one aspect, the population of cells is HLA matched with the patient. In another aspect, the population of cells is partially HLA matched or unmatched with the patient. In a further aspect, the population of cells comprises haplotyped enhanced-HSPCs.

In one aspect, the therapeutically effective amount of the population of cells comprises about 2 x 10⁶ cells to about 2 x 10¹⁰ CD34+ hematopoietic cells.

In one aspect, the method comprises more than one administration of a therapeutically effective amount of cells. In a further aspect, the frequency of administrations ranges from about every other week to about every six months. In one aspect, the initial administration is a higher number of cells than a subsequent administration.

In one aspect, the inflammatory disorder is selected from inflammation of the lungs, joints, connective tissue, eyes, nose, bowel, kidney, liver, skin, central nervous system, endocrine system, cardiovascular system and heart.

In one aspect, the inflammation of the lung is selected from asthma, adult respiratory distress syndrome, bronchitis, pulmonary inflammation, pulmonary fibrosis, and cystic fibrosis.

In one aspect, the inflammation of the joints is selected from rheumatoid arthritis, rheumatoid spondylitis, juvenile rheumatoid arthritis, osteoarthritis, gouty arthritis and other arthritic conditions.

In one aspect, the inflammation of the eye is selected from uveitis (including iritis), conjunctivitis, scleritis, keratoconjunctivitis sicca, and retinal diseases, including, but not limited to, diabetic retinopathy, retinopathy of prematurity, retinitis pigmentosa, and dry and wet age-related macular degeneration.

In one aspect, the inflammation of the bowels is selected from Crohn's disease, ulcerative colitis and distal proctitis.

In one aspect, the inflammation of the skin is selected from psoriasis, eczema and dermatitis, (e.g., eczematous dermatitides, topic and seborrheic dermatitis, allergic or irritant contact dermatitis, eczema craquelee, photoallergic dermatitis, phototoxic dermatitis, phytophotodermatitis, radiation dermatitis, and stasis dermatitis), scleroderma, ulcers and erosions resulting from trauma, burns, bullous disorders, or ischemia of the skin or mucous membranes, several forms of ichthyoses, epidermolysis bullosae, hypertrophic scars, keloids, cutaneous changes of intrinsic aging, photoaging, frictional blistering caused by mechanical shearing of the skin, cutaneous atrophy resulting from the topical use of corticosteroids, cheilitis, chapped lips, nasal irritation, mucositis and vulvovaginitis.

In one aspect, the inflammation of the endocrine system is selected from autoimmune thyroiditis (Hashimoto's disease), Type I diabetes, Type II diabetes, and acute and chronic inflammation of the adrenal cortex.

In one aspect, the inflammation of the cardiovascular system are selected from coronary infarct damage, peripheral vascular disease, myocarditis, vasculitis, revascularization of stenosis, artherosclerosis, and vascular disease associated with Type II diabetes.

In one aspect, the inflammation of the kidney is selected from glomerulonephritis, interstitial nephritis, lupus nephritis, nephritis secondary to Wegener's disease, acute renal failure secondary to acute nephritis, Goodpasture's syndrome, post-obstructive syndrome and tubular ischemia.

In one aspect, the inflammation of the liver is selected from hepatitis (arising from viral infection, autoimmune responses, drug treatments, toxins, environmental agents, or as a secondary consequence of a primary disorder), biliary atresia, primary biliary cirrhosis and primary sclerosing cholangitis.

In one aspect, the inflammation of the central nervous system is selected from multiple sclerosis and neurodegenerative diseases such as Alzheimer's disease, Parkinson's disease, or dementia associated with HIV infection.

In one aspect, the administration is systemic. In another aspect, the administration is local.

In one aspect, the administration is intravenous, intraarterial, intramuscular, intrathecal, intracapsular, intraorbital, intracardiac, intradermal, intraperitoneal, transtracheal, subcutaneous (subdermal), subcuticular, intraarticular, subcapsular, subarachnoid, intraspinal, intrastemal injection, or by infusion.

In one aspect, the method is part of a combination therapy.

A fifth object of the invention includes a pharmaceutical composition comprising a population of modulated hematopoietic cells that expresses an increased level of PD-L1 and/or IDO-1 expression that is about 3 fold to about 80 fold compared to a level of PD-L1 and/or IDO-1 expression in a population of non-modulated hematopoietic cells.

In one aspect, the pharmaceutical composition further comprises a pharmaceutically acceptable carrier.

In one aspect, population of hematopoietic cells is derived from cord blood, peripheral blood, bone marrow, or iPSCs.

In a particular aspect, the population of hematopoietic cells is derived from differentiated iPSCs.

In one aspect, the population comprises HSPCs. In a further aspect, the population comprises at least about 50% HPSCs, at least about 50%, at least about 60%, at least about 70%, at least about 80%, at least about 85%, at least about 90%, at least about 95%, at least about 98%, or at least about 99% HSPCs. In yet a further aspect, the population of hematopoietic cells comprises a substantially pure population of HSPCs.

In one aspect, the population is enriched for CD34+ HPSCs.

In one aspect, the composition is formulated for intravenous administration, intraarterial administration, intramuscular administration, intrathecal administration, intracapsular administration, intraorbital administration, intracardiac administration, intradermal administration, intraperitoneal administration, transtracheal administration, subcutaneous (subdermal) administration, subcuticular administration, intraarticular administration, subcapsular administration, subarachnoid administration, intraspinal administration, intrastemal administration, and infusion.

In one aspect, the pharmaceutical composition is formulated for a local or non-intravenous administration.

In one aspect, the population of cells comprises about 2 x 10⁶ to about 2 x 10¹⁰ CD34+ hematopoietic cells.

A sixth object of the invention includes a kit comprising the modulated cells obtained from the first object of the invention or a pharmaceutical composition according to the fifth object of the invention and a second active agent for use in a combination therapy.

### BRIEF DESCRIPTION OF THE DRAWINGS

Figs. 1A-1E show gene expression following the modulation of hematopoietic stem and progenitor cells for increased PD-L1 expression.
Figs. 2A-2D show PD-L1 surface expression on hematopoietic stem and progenitor cells following modulation for increased PD-L1 expression.
Fig. 3 shows T cell proliferation in the presence of HSC with modulated PD-L1 expression.
Fig. 4 shows gene expression following the modulation of hematopoietic stem and progenitor cells for increased PD-L1 expression.
Figs. 5A-D show IDO-1 gene expression levels in hematopoietic stem and progenitor cells following modulation for increased IDO-1 expression.
Fig. 6 shows modulation for 24 hours at 37° C with additional exogenous agents for increased IDO-1 expression.
Fig. 7 shows PD-L1 surface expression hematopoietic stem and progenitor cells following modulation for increased PD-L1 expression for 6, 24, or 48 hours.
Fig. 8 demonstrates that post-modulation, the modulated cells are viable and express PD-L1 at the cell surface when maintained in a variety of conditions.
Fig. 9A and 9B demonstrate that ex *vivo* treated human stem and progenitor cells suppress the proliferation of both autologous and allogeneic T cells.
Fig. 10 demonstrates that genetic overexpression of PD-L1 in human CD34+ stem and progenitor cells enhances suppression of T cell proliferation.
Fig. 11 demonstrates that genetic overexpression of IDO-1 in human CD34+ stem and progenitor cells enhances suppression of T cell proliferation.
Fig. 12 shows that T cells treated with media from a modulated population of hematopoietic cells demonstrating upregulated IDO-1 expression significantly suppressed T cell proliferation.

### DETAILED DESCRIPTION

### I. Definitions

The articles "a," "an," and "the" are used herein to refer to one or to more than one (i.e., to at least one) of the grammatical object of the article. By way of example, "an element" means one element or more than one element.

The use of the alternative (e.g., "or") should be understood to mean either one, both, or any combination thereof of the alternatives.

As used herein, the term "about" or "approximately" refers to a quantity, level, value, number, frequency, percentage, dimension, size, amount, weight or length that varies by as much as 15%, 10%, 9%, 8%, 7%, 6%, 5%, 4%, 3%, 2% or 1% to a reference quantity, level, value, number, frequency, percentage, dimension, size, amount, weight or length. In one embodiment, the term "about" or "approximately" refers a range of quantity, level, value, number, frequency, percentage, dimension, size, amount, weight or length ± 15%, ± 10%, ± 9%, ± 8%, ± 7%, ± 6%, ± 5%, ± 4%, ± 3%, ± 2%, or ± 1% about a reference quantity, level, value, number, frequency, percentage, dimension, size, amount, weight or length.

As used herein, the term "substantially" refers to a quantity, level, value, number, frequency, percentage, dimension, size, amount, weight or length that is 90%, 92%, 93%, 94%, 95%, 96%, 97%, 98%, 99% or higher of a reference quantity, level, value, number, frequency, percentage, dimension, size, amount, weight or length. In one embodiment, "substantially the same" refers to a quantity, level, value, number, frequency, percentage, dimension, size, amount, weight or length that produces an effect, e.g., a physiological effect, that is approximately the same as a reference quantity, level, value, number, frequency, percentage, dimension, size, amount, weight or length.

As used herein, the terms "substantially free of" and "essentially free of" are used interchangeably, and when used to describe a composition, such as a cell population or culture media, refer to a composition that is free of a specified substance, such as, 95% free, 96% free, 97% free, 98% free, 99% free of the specified substance, or is undetectable as measured by conventional means. In one embodiment, "substantially pure" may be used to denote that the composition or component is substantially free of contaminants, such as other cell types. Similar meaning can be applied to the term "absence of," where referring to the absence of a particular substance or component of a composition.

Reference throughout this specification to "one embodiment," "an embodiment," "a particular embodiment," "a related embodiment," "a certain embodiment," "an additional embodiment," or "a further embodiment" or combinations thereof means that a particular feature, structure or characteristic described in connection with the embodiment is included in at least one embodiment of the present invention. Thus, the appearances of the foregoing phrases in various places throughout this specification are not necessarily all referring to the same embodiment. Furthermore, the particular features, structures, or characteristics may be combined in any suitable manner in one or more embodiments.

Throughout this specification, unless the context requires otherwise, the words "comprise", "comprises" and "comprising" will be understood to imply the inclusion of a stated step or element or group of steps or elements but not the exclusion of any other step or element or group of steps or elements. As used herein, the terms "include" and "comprise" are used synonymously.

By "consisting of" is meant including, and limited to, whatever follows the phrase "consisting of." Thus, the phrase "consisting of" indicates that the listed elements are required or mandatory, and that no other elements may be present.

By "consisting essentially of' is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that no other elements are optional and may or may not be present depending upon whether or not they affect the activity or action of the listed elements.

The term "ex *vivo"* refers generally to activities that take place outside an organism, such as experimentation or measurements done in or on living tissue in an artificial environment outside the organism, preferably with minimum alteration of the natural conditions. In particular embodiments, "ex *vivo"* procedures involve living cells or tissues taken from an organism and cultured or modulated in a laboratory apparatus, usually under sterile conditions, and typically for a few hours or up to about 24 hours, but including up to 48 or 72 hours, depending on the circumstances. In certain embodiments, such tissues or cells can be collected and frozen, and later thawed for ex *vivo* treatment. In one embodiment, the ex *vivo* modulation is for at least about 1 hour, at least about 2 hours, at least about 3 hours, at least about 4 hours, at least about 5 hours, at least about 6 hours, at least about 7 hours, at least about 8 hours, at least about 9 hours, at least about 12 hours, at least about 18 hours, at least about 24 hours, or at least about 36 hours. In one embodiment, the ex *vivo* modulation is for a time period ranging from time period ranging from about 1 hour to about 72 hours, about 2 hours to about 48 hours, about 4 hours to about 48 hours, about 6 hours to about 48 hours, about 12 hours to about 48 hours, about 1 hour to about 24 hours, about 2 hours to about 24 hours, about 4 hours to about 24 hours, about 6 hours to about 24 hours, about 12 hours to about 24 hours, about 1 hour to about 12 hours, about 4 hours to about 12 hours, about 6 hours to about 12 hours, or about 8 hours to about 12 hours. Tissue culture experiments or procedures lasting longer than a few days using living cells or tissue are typically considered to be *"in vitro,"* though in certain embodiments, this term can be used interchangeably with *ex vivo.*

The term *"in vivo"* refers generally to activities that take place inside an organism.

The recitations "*ex vivo* administration," "*ex vivo* treatment," or "*ex vivo* modulation," relate generally to medical procedures in which one or more organs, cells, or tissues are obtained from a living or recently deceased subject, optionally purified/enriched, exposed to a treatment or procedure (e.g., an *ex vivo* administration step that involves incubating the cells with a composition or agent of the present invention to enhance engraftment of particular cells, such as hematopoietic stem or progenitor cells). Cells treated ex *vivo* may be administered to the donor or to a different living subject.

Such *ex vivo* therapeutic applications may also include an optional *in vivo* treatment or procedural step, such as by administering cells with therapeutic potential one or more times to a living subject. Both local and systemic administration is contemplated for these embodiments, according to well-known techniques in the art and as described elsewhere herein. The amount of therapeutic cells administered to a subject will depend on the characteristics of that subject, such as general health, age, sex, body weight, and tolerance to drugs, as well as the degree, severity, and type of reaction to the drug and/or cell transplant.

As used herein, the term "incubating" is used to describe a specific step or steps by which cells or populations of cells are manipulated. Incubation steps may include specific temperatures, agents, or conditions which modulate the cell or populations of cells.

As used herein, the term "exogenous" is used interchangeably with the term "heterologous" refer to a substance coming from some source other than its native source. For example, the terms "exogenous protein," or "exogenous cell" refer to a protein or cell from a non-native source or location, and that have been artificially supplied to a biological system. In contrast, the terms "endogenous protein," or "endogenous cell" refer to a protein or cell that are native to the biological system, species or individual.

The phrase "stem cell" as used herein refers to a cell which is an undifferentiated cell capable of (1) long term self-renewal, or the ability to generate at least one identical copy of the original cell, (2) differentiation at the single cell level into multiple, and in some instance only one, specialized cell type and (3) of *in vivo* functional regeneration of tissues. Stem cells are subclassified according to their developmental potential as totipotent, pluripotent, multipotent and oligo/unipotent. A "progenitor cell" also has the capacity to self-renew and to differentiate into more mature cells, but is committed to a lineage (e.g., hematopoietic progenitors are committed to the blood lineage; myeloid progenitors are committed to the myeloid lineage; lymphoid progenitors are committed to the lymphoid lineage), whereas stem cells are not necessarily so limited. "Self-renewal" refers a cell with a unique capacity to produce unaltered daughter cells and therefore replenish and maintain its population numbers, and to generate specialized cell types (potency). Self-renewal can be achieved in two ways. Asymmetric cell division produces one daughter cell that is identical to the parental cell and one daughter cell that is different from the parental cell and is a more committed progenitor or differentiated cell. Symmetric cell division produces two identical daughter cells. "Proliferation" or "expansion" of cells refers to symmetrically dividing cells.

As used herein, the term "progenitor" or "progenitor cells" refers to cells that have the capacity to self-renew and to differentiate into more mature cells. Progenitor cells have a reduced potency compared to pluripotent and multipotent stem cells. Many progenitor cells differentiate along a single lineage, but may also have quite extensive proliferative capacity.

As used herein, the term "hematopoietic stem and progenitor cell" or "HSPC" refers to a cell identified by the presence of the antigenic marker CD34 (CD34+) and are therefore characterized as CD34+ cells, and populations of such cells. In particular embodiments, the term "HSPC" refers to a cell identified by the presence of the antigenic marker CD34 (CD34+) and the absence of lineage (Lin) markers and are therefore characterized as CD34+/Lin(-) cells, and populations of such cells. It is recognized that the population of cells comprising CD34+ and/or Lin(-) cells also includes hematopoietic progenitor cells. The term "hematopoietic cell" refers to a continuum of cells ranging from a HSPC, to a fully differentiated blood cell, including all the cells of the myeloid and lymphoid lineages.

As used herein, the term "induced pluripotent stem cells" or, iPSCs, means that the stem cells are produced from differentiated adult, neonatal or fetal cells that have been induced or changed, i.e., reprogrammed into cells capable of differentiating into tissues of all three germ or dermal layers: mesoderm, endoderm, and ectoderm. The iPSCs produced do not refer to cells as they are found in nature.

As used herein, a "non-contacted," "non-treated," or an "untreated" cell is a cell that has not been treated, e.g., cultured, contacted, or incubated with an agent other than a control agent. Cells contacted with DMSO (a control agent), or contacted with another vehicle are non-contacted cells.

As used herein, the term "isolated" refers to material that is removed from its original environment. For example, an "isolated population of cells," an "isolated source of cells," or "isolated HSPCs" and the like, as used herein, refer to *in vitro* or *ex vivo* separation of one or more cells from their natural cellular environment, and from association with other components of the tissue or organ, i.e., it is not significantly associated with *in vivo* substances.

As used herein, the terms "agent," and "exogenous agent," are used interchangeably to refer to a compound or molecule capable of increasing expression of PD-L1 and/or IDO-1 in a cell that is contacted with the agent.

As used herein, the term "subject" refers to any animal, preferably a human patient, livestock, or other domesticated animal.

As used herein, the terms "treatment," "treating," and the like, refer to obtaining a desired pharmacologic and/or physiologic effect, including without limitation achieving an improvement or elimination of symptoms of a disease. The effect may be prophylactic in terms of completely or partially preventing a disease or symptom thereof and/or may be therapeutic in terms of achieving an improvement or elimination of symptoms, or providing a partial or complete cure for a disease and/or adverse effect attributable to the disease. "Treatment," as used herein, covers any treatment of a disease in a mammal, particularly in a human, and includes: (a) preventing the disease from occurring in a subject which may be predisposed to the disease but has not yet been diagnosed as having it; (b) inhibiting the disease, i.e., arresting its development; (c) relieving the disease, e.g., causing regression of the disease, e.g., to completely or partially eliminate symptoms of the disease; and (d) restoring the individual to a pre-disease state, e.g., reconstituting the hematopoietic system.

The terms "enhance," "promote," "increase" and "activate" refer generally to the ability of an agent to produce or cause a greater physiological response (i.e., downstream effects) in a cell, as compared to the response caused by either vehicle or a control molecule/composition, e.g., increased PD-L1 and/or IDO-1 expression in a cell, such as for example, a hematopoietic stem and progenitor cell. A measurable physiological response may include an increase in the ability of a cell to modulate an immune response in a subject. An "increased" or "enhanced" amount is typically a "statistically significant" amount, and may include an increase that is 1.1, 1.2, 1.5, 2, 3, 4, 5, 6, 7, 8, 9, 10, 15, 20, 30 or more times (e.g., 500, 1000 times) (including all integers and decimal points in between and above 1, e.g., 1.5, 1.6, 1.7. 1.8, etc.) the response produced by vehicle (the absence of an agent) or a control composition.

The terms "decrease," "lower," "lessen," "reduce," and "abate" refer generally to the ability of an agent to produce or cause a lesser physiological response (i.e., downstream effects) in a cell, as compared to the response caused by either vehicle or a control molecule/composition. A "decreased" or "reduced" amount is typically a "statistically significant" amount, and may include an decrease that is 1.1, 1.2, 1.5, 2, 3, 4, 5, 6, 7, 8, 9, 10, 15, 20, 30 or more times (e.g., 500, 1000 times) (including all integers and decimal points in between and above 1, e.g., 1.5, 1.6, 1.7, 1.8, etc.) the response produced by vehicle (the absence of an agent) or a control composition.

The "therapeutic potential" of a cell refers to the therapeutic quality of the cell, the cell's ability to provide a therapeutic benefit when administered to a subject. In particular embodiments, the therapeutic potential of a cell can be measured, quantified, determined, identified, or validated by increased expression of PD-L1 and/or IDO-1. Therapeutic potential includes, but is not limited to, a cell's ability to inhibit an immune response in a subject.

The term "gene" means the segment of DNA involved in producing a polypeptide chain; it includes regions preceding and following the coding region (leader and trailer) as well as intervening sequences (introns) between individual coding segments (exons).

"Gene expression," or "expression," as used herein refers to the transcription of a gene in the production of RNA (e.g., mRNA, rRNA, tRNA, miRNA, or snRNA), as well as the translation of an mRNA in the production of a protein, or the relative levels of the translated product of a transcribed gene. Gene expression and/or the pattern of expression of a gene may be detected in a biological sample, such as hematopoietic cells, stem and progenitor cells, or a population of cells comprising stem or progenitor cells, including, but not limited to, hematopoietic stem and progenitor cells.

An "expression vector" is a nucleic acid construct, generated recombinantly or synthetically, with a series of specified nucleic acid elements that permit transcription of a particular nucleic acid in a host cell. The expression vector can be part of a plasmid, virus, or nucleic acid fragment. Typically, the expression vector includes a nucleic acid to be transcribed operably linked to a promoter.

The term "PD-L1" refers to programmed death-ligand 1, the 40kDa type 1 transmembrane protein that is encoded by the CD274 gene. PD-L1 binds to its receptor, PD-1, found on activated T cells, B cells, and myeloid cells. PD-L1 is also known as "CD274," "B7 homolog 1," and "B7-H1."

"IDO-1" refers to indoleamine 2,3-dioxygenase, the 46kDA enzyme catalyzing the oxidative catabolism of the essential amino acid tryptophan (TRP) and producing kynurenine (KYN) pathway metabolites.

The term "modulate," or "modulation," as used herein refers to a change in the cells' physiological status or modify or alter the properties of a cell. For instance, increasing the expression of a desired target gene, such as PD- L1 and/or IDO-1, or increasing or decreasing an immune response from a cell.

### II. Cell Modulation

The invention provides compositions and methods to modulate the immune system through the immuno-regulatory properties of cells expressing increased levels of programmed death ligand 1 (PD-L1) and indoleamine 2,3-dioxygenase 1 (IDO-1). The invention generally relates to methods and compositions for modulating cells with exogenous agents to achieve an increase in PD-L1 and/or IDO-1 expression in the cells. The invention also relates to cells having increased PD-L1 and/or IDO-1 expression, and methods of using such cells in the therapeutic applications, including the treatment of immunological disorders and inflammation.

Some aspects of the invention relate to the conditions for modulating a cell to achieve an increase in PD-L1 and/or IDO-1 expression. In some embodiments, such conditions comprise contacting the cell with one or more exogenous agents capable of increasing PD-L1 and/or IDO-1 expression in the cell. In one embodiment, the cell is contacted with at least two or at least three exogenous agents.

Such contact may occur, for example, by incubating the cell in the presence of one or more exogenous agents capable of increasing PD-L1 and/or IDO-1 expression in the cell. Conditions for contacting the cell may occur *in vitro* or *ex vivo,* such as under standard culture conditions, for example.

Another aspect of the invention relates to the time period during which the cell is contacted with the one or more agents capable of increasing PD-L1 and/or IDO-1 expression. In some embodiments, the cell is contacted with the one or more agents for a time of between about 5 minutes to about 96 hours. In other embodiments, the cell is contacted with the one or more agents for about 1 hour, about 2 hours, about 3 hours, about 5 hours, about 10 hours, about 24 hours, about 48 hours, about 96 hours, or any time period intervening these specifically referenced times. In one embodiment, the cell is contacted for about four hours to about 48 hours. In some aspects of the invention, the cell is expanded in the presence of one or more agents capable of increasing PD-L1 and/or IDO-1 expression. Another aspect of the invention relates to the temperature at which the cell is modulated with the at least one exogenous agent. Accordingly, the cell may be modulated at any temperature that results in an increase in the expression of PD-L1 and/or IDO-1 in the cell. In some non-limiting embodiments of the invention, the cell is modulated at a temperature of between about 4°C to about 37°C. In one embodiment, the cell is modulated at about 37°C.

One aspect of the invention relates to the quantitative increase in PD-L1 and/or IDO-1 expression in the cells that results from being modulated with one or more agents capable of increasing PD-L1 and/or IDO-1 expression. It is contemplated that such increase may be about 10%, about 20%, about 30%, about 40%, about 50%, about 60%, about 70%, about 80%, about 90%, about 100%, about 150%, about 200% or more, than the cells prior to treatment. In particular embodiments, cells having increased PD-L1 and/or IDO-1 expression have been modulated under conditions sufficient to increase PD-L1 and/or IDO-1 expression at least 2, 3, 5, 10, 20, 30, 40, 50, 60, 70, or 80 fold or more in the modulated cells compared to control cells. Such increases may relate to an increase in gene expression, or an increase in protein expression.

Suitable methods for determining the level of gene expression in a sample include, but are not limited to, nucleic acid amplification, for example, by RT-PCR (U.S. Pat. No. 4,683,202), ligase chain reaction (Barany, Proc. Natl. Acad. Sci. USA 88:189-93, 1991), self-sustained sequence replication (Guatelli et al., Proc. Natl. Acad. Sci. USA 87:1874-78, 1990), transcriptional amplification system (Kwoh et al., Proc. Natl. Acad. Sci. USA 86:1173-77, 1989), Q-Beta Replicase (Lizardi et al., Bio/Technology 6:1197, 1988), rolling circle replication (U.S. Pat. No. 5,854,033), or any other nucleic acid amplification method, followed by the detection of the amplified molecules using techniques well known to those of skill in the art.

As used herein, the terms "conditions sufficient," or "under conditions sufficient," refer to the conditions for treating cells with one or more agents to increase PD-L1 and/or IDO-1 expression in the cells to surprising and unexpected levels compared to control, vehicle, or non-treated cells. Conditions include, but are not limited to the agents used to treat the cells and concentrations of agent(s), the time the cells are exposed to the agent(s), and the temperature of treatment.

### A. Modulating Agents

An aspect of the invention relates to the agents that are used to modulate cells for increased PD- L1 expression. Such agents include, but are not limited to, polynucleotides, polypeptides, small molecules, or a combination thereof. Small molecules for modulating cells include, but are not limited to, glucocorticoids and/or prostaglandin pathway agonists antineoplastics, dopamine receptor agonists and other agents.

### i. Peptides

### a. Interferon Receptor Agonists

In some aspects of the invention, cells are modulated by being contacted with one or more interferon receptor agonists. Suitable interferon receptor agonists for use with the invention include, but are not limited to, any naturally occurring or non-naturally occurring ligand of Type I, Type II or Type III interferon receptors, which binds to and causes signal transduction via the receptor. Such interferon receptor agonists include interferons, including naturally-occurring interferons, modified interferons, synthetic interferons, pegylated interferons, fusion proteins comprising an interferon and a heterologous protein, shuffled interferons, an antibody or antibodies specific for an interferon receptor, non-peptide chemical agonists, and the like.

In some aspects of the invention, the interferon receptor agonist comprises an interferon selected from the group consisting of IFN-α, IFN-β, IFN-ε, IFN-κ, IFN-ω, IFN-γ, or a combination thereof. In one non-limiting embodiment of the invention, cells are modulated with IFN-β. In another non-limiting embodiment of the invention, the cells are modulated with IFN-γ. In yet another embodiment, the cells are modulated with IFN-β and IFN-γ.

### b. Polynucleotides Encoding Peptides

In some aspects of the invention, cells are modulated with a polynucleotide comprising poly (I:C). Poly (I:C), also known as polyinosinic polycytidylic acid, is a synthetic double-stranded RNA consisting of a strand of polyriboinosinic acid (Poly I) and a strand of polyribocytidylic acid (Poly C). Poly (I:C) is known to interact with toll-like receptor 3 (TLR3) which is expressed in the membrane of B-cells, macrophages and dendritic cells. Commercial sources of poly (I:C) for use with the invention include, but are not limited to, Invivogen™ (CAS number 31852-29-6); Tocris™ (CAS number 24939-03-5), GE Healthcare Life Sciences™ (Product Code 27-4732-01), and Sigma-Aldrich™ (CAS Number 42424-50-0).

In some aspects of the invention, cells are modulated with one or more polynucleotides capable of increasing PD-L1 and/or IDO-1 expression. Suitable polynucleotides include exogenous polynucleotides that encode one or more functional PD-L1 polypeptides. Suitable polynucleotides may include exogenous polynucleotides that encode one or more functional IDO-1 polypeptides. Such polynucleotides may form part of an expression cassette which is used to genetically modify the cell to express an exogenous PD- L1 or IDO-1 polypeptide. Polynucleotides for use with the invention include, but are not limited to, those that encode human, mouse, rabbit, rat, bovine, horse, goat or non-human primate PD-L1 or IDO-1 polypeptides.

In one embodiment, the expression cassette is included in a vector. Thus, in one aspect, the cells are modulated with a vector comprising one or more polynucleotides capable of increasing PD-L1 and/or IDO-1 expression. Examples of vectors used for such purposes include expression plasmids capable of directing the expression of the nucleic acids in the target cell. In other instances, the vector is a viral vector system wherein the nucleic acids are incorporated into a viral genome that is capable of transfecting the target cell. In a preferred embodiment, the polynucleotides can be operably linked to expression and control sequences that can direct expression of the gene in the desired target host cells. Thus, one can achieve expression of the nucleic acid under appropriate conditions in the target cell.

Viral vector systems useful in the expression of the present nucleic acids include, for example, naturally occurring or recombinant viral vector systems. Depending upon the particular application, suitable viral vectors include replication competent, replication deficient, and conditionally replicating viral vectors. For example, viral vectors can be derived from the genome of human or bovine adenoviruses, vaccinia virus, herpes virus, adeno-associated virus, minute virus of mice (MVM), HIV, sindbis virus, and retroviruses (including but not limited to Rous sarcoma virus), Sendai Virus, and MoMLV. Typically, the genes of interest are inserted into such vectors to allow packaging of the gene construct, typically with accompanying viral DNA, followed by infection of a sensitive host cell and expression of the gene of interest. Accordingly, in one embodiment, the cells to be modulated are contacted with a viral vector comprising one or more polynucleotides capable of increasing PD-L1 and/or IDO-1 expression.

### ii. Prostaglandin Pathway Agonists

In some aspects of the invention, cells are modulated to increase PD-L1 and/or IDO-1 expression by contact with one or more prostaglandin pathway agonists. Such prostaglandin pathway agonists include, but are not limited to, cAMP analogues or enhancers, Gα-s activators, compounds that selectively bind the PGE₂ EP₂ or the PGE₂ EP₄ receptor, glucocorticoids, and combinations thereof.

As used herein, the term "prostaglandin pathway agonist" refers to an agent that stimulates prostaglandin cell signaling pathways, including an agent that stimulates the PGE₂R₂ and/or PGE₂R₄ cell signaling pathways. Illustrative examples of prostaglandin pathway agonists that are suitable for use in modulating cells according to the invention, include, but are not limited to PGE₂, dmPGE₂, 15(S)-15-methyl PGE₂, 20-ethyl PGE₂, 8-iso-16-cyclohexyl-tetranor PGE₂, and PGE₂ analogues. In certain embodiments, PGE₂R₂ and PGE₂R₄ agonists and analogues thereof are of particular interest, and in some embodiments, the agent preferentially binds and activates a PGE₂ EP₂ or PGE₂ EP₄ receptor.

As used herein, the terms "prostaglandin E₂" or "PGE₂" include, without limitation, any naturally-occurring or chemically synthesized PGE₂ molecule, as well as "analogues" thereof. As used herein, the term "analogue" or relates to a chemical molecule that is similar to another chemical substance, e.g., PGE₂, in structure and function, often differing structurally by a single element or group, but may differ by modification of more than one group (e.g., 2, 3, or 4 groups) if it retains the same function as the parental chemical.

Illustrative examples of PGE₂ "analogues" include, without limitation, 16,16-dimethyl PGE₂ ("dmPGE2"), p-(p-acetamidobenzamido) phenyl ester, 11-deoxy-16, 16-dimethyl PGE₂, 9-deoxy- 9-methylene-16, 16-dimethyl PGE₂, 9-deoxy-9-methylene PGE₂, 9-keto Fluprostenol, 5-trans PGE₂, 17-phenyl-omega-trinor PGE₂, PGE₂ serinol amide, PGE₂ methyl ester, 16-phenyl tetranor PGE₂, 15(S)-15-methyl PGE₂, 15(R)-15-methyl PGE₂, 8-iso-15-keto PGE₂, 8-iso PGE₂ isopropyl ester, 8-iso-16-cyclohexyl-tetranor PGE₂, 20-hydroxy PGE₂, 20-ethyl PGE₂, 11-deoxy PGE₁, nocloprost, sulprostone, butaprost, 15-keto PGE₂, and 19 (R) hydroxy PGE₂. Also included are prostaglandin analogues having a similar structure to PGE₂ that are substituted with halogen at the 9-position (see, e.g., WO 2001112596, herein incorporated by reference in its entirety), as well as 2-decarboxy-2-phosphinico prostaglandin derivatives, such as those described in U.S. Publication No. 2006/0247214, herein incorporated by reference in its entirety).

PGE₁ analogues, including without limitation alprostadil, can also be used to activate the PGE₂R₂ (EP₂) and PGE₂R₄ (EP₄) cell signaling pathways, and are contemplated as agents useful in the methods of the invention.

Without being limited to any particular theory or mechanism, stimulation/activation of the PGE₂R₂ (EP₂) and PGE₂R₄ (EP₄) cell signaling pathways result in an increase in expression of PD-L1 and/or IDO-1. Accordingly, in one embodiment, a "non-PGE₂-based ligand" that binds to and stimulates PGE₂R₂ and PGE₂R₄ receptors (i.e., a PGE₂R₂/PGE₂R₄ agonist) is contemplated for use in the methods of the invention. Illustrative examples of non-PGE₂-based EP₂ receptor agonists include CAY10399, ON0O_8815Ly, ONO-AE1-259, CP-533,536 and carbazoles and fluorenes disclosed in WO 2007/071456.

Illustrative examples of non-PGE₂-based EP₄ agonists include ONO-4819, APS-999 Na, AH23848, ONO-AE1-329, and other non-PGE₂-based EP₄ agonists disclosed in WO/2000/038663; U.S. Patent No. 6,747,037; and U.S. Patent No. 6,610,719).

In some aspects of the invention, cells are modulated with agents that are selective for, and preferentially bind to, PGE₂ EP₄ receptors. Such agents have a higher affinity for the EP₄ receptor than for any of the other three EP receptors namely EP₁, EP₂ and EP₃. Agents that selectively bind the PGE EP₄ receptor include, but are not limited to, agents selected from the group consisting of: 5-[(1E,3R)-4,4-difluoro-3-hydroxy-4-phenyl-l-buten-l-yl]-l-[6-(2H-tetrazol-5R-yl)hexyl]-2-pyrrolidinone; 2-[3-[(1R,2S,3R)- 3 -hydroxy-2- [(E,3 S)-3 -hydroxy-5 - [2-(methoxymethyl)phenyl]pent- 1 -enyl] -5 - oxocyclopentyljsulfanylpropylsulfanyl] acetic acid; methyl 4-[2-[(1R,2R,3R)-3-hydroxy-2- [(E,3 S)-3 -hydroxy-4- [3 -(methoxymethyl)phenyl]but- 1-enyl]-5 - oxocyclopentyl]ethylsulfanyl]butanoate; 16-(3-Methoxymethyl)phenyl-ro-tetranor-5-thiaPGE; 5- {3-[(2S)-2-{(3R)-3-hydroxy-4-[3-(trifluoromethyl)phenyl]butyl} -5-oxopyrrolidin-1 - yl]propyl]thiophene -2-carboxylate; [4' -[3-butyl-5-oxo-1-(2-trifluoromethyl-phenyl)-1,5-dihydro-[1 ,2,4]triazol-4-ylmethyl]-biphenyl-2-sulfonicacid (3-methyl-thiophene-2-carbonyl)- amide]; and ((Z)-7- {(1 R,4S,5R)-5-[(E)-5-(3-chloro-benzo[b]thiophene-2-yl)-3-hyd roxy-pent- 1- enyl]-4-hydroxy-3,3-dimethyl-2-oxo-cyclopentyl}-hept-5-enoic acid), and pharmaceutically acceptable salts of any of these agents.

In particular embodiments, the prostaglandin pathway agonist comprises PGE₂, 16,16-dmPGE₂, 15(S)-15-methyl PGE₂, 20-ethyl PGE₂, or 8-iso-16-cyclohexyl-tetranor PGE₂.

### iii. Glucocorticoids

In some aspects of the invention, cells are modulated for increased PD-L1 and/or IDO-1 expression by contact with glucocorticoids and/or glucocorticoid receptor agonists.

Illustrative examples of glucocorticoids and glucocorticoid receptor agonists suitable for use with the invention include, but are not limited to, medrysone, alclometasone, alclometasone dipropionate, amcinonide, beclometasone, beclomethasone dipropionate, betamethasone, betamethasone benzoate, betamethasone valerate, budesonide, ciclesonide, clobetasol, clobetasol butyrate, clobetasol propionate, clobetasone, clocortolone, cloprednol, cortisol, cortisone, cortivazol, deflazacort, desonide, desoximetasone, desoxycortone, desoxymethasone, dexamethasone, diflorasone, diflorasone diacetate, diflucortolone, diflucortolone valerate, difluorocortolone, difluprednate, fluclorolone, fluclorolone acetonide, fludroxycortide, flumetasone, flumethasone, flumethasone pivalate, flunisolide, flunisolide hemihydrate, fluocinolone, fluocinolone acetonide, fluocinonide, fluocortin, fluocoritin butyl, fluocortolone, fluorocortisone, fluorometholone, fluperolone, fluprednidene, fluprednidene acetate, fluprednisolone, fluticasone, fluticasone propionate, formocortal, halcinonide, halometasone, hydrocortisone, hydrocortisone acetate, hydrocortisone aceponate, hydrocortisone buteprate, hydrocortisone butyrate, loteprednol, meprednisone, 6a-methylprednisolone, methylprednisolone, methylprednisolone acetate, methylprednisolone aceponate, mometasone, mometasone furoate, mometasone furoate monohydrate, paramethasone, prednicarbate, prednisolone, prednisone, prednylidene, rimexolone, tixocortol, triamcinolone, triamcinolone acetonide and ulobetasol, as well as combinations thereof.

In particular embodiments, the glucocorticoid comprises betamethasone, clobetasol proprionate, flumethasone, flucinolone acetonide, medrysone, hydrocortisone, triamcinolone, alclometasone, or dexamethasone. In more particular embodiments, the glucocorticoid is medrysone.

### iv. Other Agents

Other agents of particular interest with IDO-1 include antineoplastics (such as gemcitabine, letrozole, and fludarabine), dopamine receptor antagonists (e.g., fluphernazine), and various others such as isometheptene mucate, dihydrostreptomycin sulfate, protriptyline, telenzepine, cyclobenzaprine, and 4-aminosalicylic acid.

### B. Cells

Aspects of the invention relate to the cells which are modulated to achieve increased PD-L1 and/or IDO-1 expression. Accordingly, the invention may be practiced with any cell or combination of cells that responds to modulation with one or more agents capable of increasing PD-L1 expression in the cell or combination of cells.

Cells for use with the invention may be autologous, allogeneic, syngeneic or xenogeneic with respect to the subject to which they are administered. "Autologous," as used herein, refers to cells from the same subject. "Allogeneic," as used herein, refers to cells of the same species that differ genetically to the cell in comparison. "Syngeneic," as used herein, refers to cells of a different subject that are genetically identical to the cell in comparison. "Xenogeneic," as used herein, refers to cells of a different species to the cell in comparison. In preferred embodiments, the cells of the invention are allogeneic.

Cells for use with the invention include, but are not limited to stem cells, progenitor cells, and differentiated cells. The stem cells described herein may comprise embryonic stem cells, induced pluripotent stem cells, bone marrow stem cells, umbilical cord stem cells, placental stem cells, mesenchymal stem cells, neural stem cells, liver stem cells, pancreatic stem cells, cardiac stem cells, T cells, kidney stem cells, hematopoietic stem cells and muscle stem cells. The cells may be obtained from a tissue explant, a primary culture of cells, clonal cells, or serially expanded cells.

The cells may be present in a cell population. Cell populations include whole blood samples, e.g., whole cord blood, whole mobilized peripheral blood, whole bone marrow samples; isolated cells expressing particular markers, e.g., CD34+; and hematopoietic stem and progenitor cells. Suitable sources of cells for use in the methods of the present invention include, but are not limited to, cells isolated or obtained from an organ or tissue of the body containing cells of hematopoietic origin. By "isolated" is meant material that is removed from its original environment. For example, a cell is isolated if it is separated from some or all of the components that normally accompany it in its native state. For example, an "isolated population of cells," an "isolated source of cells," or "isolated hematopoietic stem cells" and the like, as used herein, refer to *in vitro* or ex *vivo* separation of one or more cells from their natural cellular environment, and from association with other components of the tissue or organ, e.g., it is not significantly associated with *in vivo* substances.

Populations of cells described herein can be obtained from bone marrow, umbilical cord blood, mobilized peripheral blood, Wharton's jelly, placenta, fetal blood, or obtained from an induced pluripotent stem cell (iPSC).

In one embodiment, the present compositions and methods may use a "hematopoietic cell," e.g., a cell selected from the continuum of cells ranging from a HSPC, to a fully differentiated blood cell, including all the cells of the myeloid and lymphoid lineages. Thus, in one embodiment, a suitable source of cells for use in the methods of the present invention includes, but is not limited to, cells isolated or obtained from an organ or tissue of the body containing cells of hematopoietic origin. In another embodiment, the cell may be obtained from an iPSC or a population of iPSCs, where the iPSC(s) are differentiated to form a hematopoietic cell.

Thus, hematopoietic cells for modulation and use in the methods described herein can be obtained from bone marrow. Hematopoietic cells for modulation and use in the methods described herein can be obtained from umbilical cord blood. Hematopoietic cells for modulation and use in the methods described herein can be obtained from mobilized peripheral blood. Hematopoietic cells for modulation and use in the methods described herein can be obtained from Wharton's jelly. Hematopoietic cells for use in the methods described herein can be obtained from placenta. Hematopoietic cells for modulation and use in the methods described herein can be obtained from fetal blood.

Hematopoietic cells described herein can be obtained or isolated from unfractionated or fractioned bone marrow of adults, which includes femurs, hip (*e.g*. iliac crest), ribs, sternum, or any other bone containing marrow. Hematopoietic cells described herein can be obtained or isolated directly by removal from the hip using a needle and syringe, or from the blood, often following pre-treatment with cytokines, such as G-CSF (granulocyte colony-stimulating factors), that induce cells to be released or mobilized from the bone marrow compartment. Other sources of hematopoietic cells described herein include umbilical cord blood, placental blood, and mobilized peripheral blood.

The hematopoietic cells described herein can be harvested (*e.g*., isolated) from a hematopoietic source, *e.g*., bone marrow cells, umbilical cord blood, or mobilized peripheral blood cells. "Harvesting" hematopoietic stem and progenitor cells is defined as the dislodging or separation of cells from the matrix. This can be accomplished using a number of methods known in the art including, for example, enzymatic, non-enzymatic, centrifugal, electrical, or size-based methods, or preferably, by flushing the cells using media (*e.g*., media in which the cells are incubated). In particular embodiments, harvesting a sufficient quantity of cells for transplantation can be obtained by mobilizing the stem and progenitor cells in the donor.

In some aspects of the invention, HPSCs are obtained from mobilized peripheral blood. "Hematopoietic stem cell mobilization" refers to the release of stem cells from the bone marrow into the peripheral blood circulation for the purpose of leukapheresis, prior to transplantation. Hematopoietic growth factors, e.g., granulocyte colony stimulating factor (G-CSF) or chemotherapeutic agents often are used to stimulate the mobilization. Commercial stem cell mobilization drugs, e.g., MOZOBIL™, can be used in combination with G-CSF to mobilize sufficient quantities of HPSCs for transplantation into a subject. Mobilized peripheral blood may be obtained by treating a donor with an agent that promotes recruitment of hematopoietic stem/progenitor cells (HPSC) from the bone marrow into peripheral blood. Suitable agents and methods for mobilizing peripheral blood for use in the invention include, but are not limited to, those disclosed in the following references, the disclosure of which are incorporated by reference in their entirety: Lemoli et al., Haematologica, 2008 Mar: 93:321-324; Pelus, Curr Opin Hematol. 2008 Jul;15(4):285-92; and US 2012/0003189.

Hematopoietic cells for use in the therapeutic compositions and methods of the invention can be obtained from pluripotent stem cell sources (*e.g*., induced pluripotent stem cells (iPSCs) and embryonic stem cells (ESCs)). As used herein, the term "induced pluripotent stem cell" or "iPSC" refers to a non-pluripotent cell that has been reprogrammed to a pluripotent state. Once the cells of a subject have been reprogrammed to a pluripotent state, the cells can then be differentiated to a desired cell type, such as a hematopoietic stem or progenitor cell. As used herein, the terms "reprogramming" refers to a method of increasing the potency of a cell to a less differentiated state. Suitable methods and materials for reprogramming a cell (*e.g*. somatic cell) to an iPSC include, but are not limited to, those disclosed in the following documents, the entire contents of which are incorporated by reference: U.S. Patent Publication No. 2011/0076678, U.S. Patent Publication No. 2013/0102074, U.S. Patent Publication No. 2010/0310525, U.S. Patent Publication No. 2011/0110899, U.S. Patent Publication No. 2007/0254884, US 2012/0028351, U.S. Patent Publication No. 2012/0264218, U.S. Patent No. 8,932,856, Yamanaka et al. Cell. 2006 Aug 25;126(4):663-76, Zhou et al. Cell Stem Cell Stem Cell. 2009 May 8;4(5):381-4; Wemig et al. Nature. 2007 Jul 19;448(7151):318-24; Okita et al. Science. 2008 Nov 7;322(5903):949-53; Woltjen et al. Nature. 2009 Apr 9;458(7239):766-70, U.S. Patent Publication No. 2010/0233804; U.S. Patent Publication No. 2012/0264218; U.S. Patent No. 7,592,177; U.S. Patent No. 7,951,592; U.S. Patent No. 8,071,369; U.S. Patent No. 8,309,555; and U.S. Patent No. 8,906,677.

Hematopoietic cells described herein can be purified using techniques well known in the art. For example, the human hematopoietic cells (for instance HSPCs) described herein can be purified using FACS or flow cytometry as understood in the art and exemplified in, for example, U.S. Ser. No. 13/257,290 (US 20120202288), which is herein incorporated in full by reference. HSPCs for use with the invention may also be derived from a clonal cell line.

Hematopoietic cells described herein, whether obtained from cord blood, bone marrow, peripheral blood, or other source, can be grown, treated or expanded in any suitable, commercially available or custom defined medium, with or without serum, as desired (see, e.g., Hartshorn et al., Cell Technology for Cell Products, pages 221-224, R. Smith, Editor; Springer Netherlands, 2007, herein incorporated by reference in its entirety). For instance, in certain embodiments, serum free medium can utilize albumin and/or transferrin, which can be useful for the growth and expansion of, for example, CD34+ cells in serum free medium. Cytokines can be included, such as, but not limited to, Flt-3 ligand, stem cell factor (SCF), thrombopoietin (TPO), and IL-6, among others. Hematopoietic cells (for instance HSPCs) can be grown in vessels such as bioreactors (see, *e.g*., Liu et al., Journal of Biotechnology 124:592-601, 2006, herein incorporated by reference in its entirety). A suitable medium for *ex-vivo* expansion of HSPCs can include HSPC supporting cells, such as stromal cells (*e.g*., lymphoreticular stromal cells). Stromal cells can be derived, for instance, from the disaggregation of lymphoid tissue. Stromal cells can support the *in vitro, ex vivo,* and *in vivo* maintenance, growth, and differentiation of HSPCs, as well as their progeny.

When transplanted into irradiated humans, hematopoietic cells described herein (e.g., modulated hematopoietic cells) can repopulate the erythroid, neutrophil-macrophage, megakaryocyte, and lymphoid hematopoietic cell pool. HSPCs described herein can be identified according to certain phenotypic or genotypic markers. For example, HSPCs can be identified by their small size, lack of lineage (lin) markers, low staining (side population) with vital dyes such as rhodamine 123 (rhodamine*^{DULL}*, also called rho*^{lo}*) or Hoechst 33342, and by the presence of various antigenic markers on their surface, many of which belong to the cluster of differentiation series (*e.g*., CD34, CD38, CD90, CD133, CD105, and c-kit, the receptor for stem cell factor). In some aspects, HSPCs are CD34+ cells. HSPCs described herein can be considered negative for the markers that are typically used to detect lineage commitment, and, thus, are often referred to as Lin(-) cells. Most human HSPCs (*e.g*. hHSPCs) can be characterized as CD34+, CD59+, Thy1/CD90+, CD38101-, C-kit/CD117+, and Lin(-). However, not all stem cells are covered by these combinations, as certain HSPCs are CD34-/CD38-. Also some studies suggest that earliest stem cells can lack c-kit on the cell surface. For HSPCs, CD133 can represent an early marker. CD34+ and CD34-HSPCs in certain instances have been shown to be CD133+. CD34+ and Lin(-) cells can also include hematopoietic progenitor cells.

The cells described herein can be HLA typed and can be matched or partially matched to a specific subject for the administration. Alternatively, the cells may be unmatched to a specific subject for administration. HLA-type refers to the unique set of proteins called human leukocyte antigens. These proteins are present on each individual's cells and allow the immune system to recognize 'self from 'foreign'. Administration of cells or tissues that are recognized as foreign can lead to compatibility problems including, for example, immunorejection or graft versus host disease (GVHD). There are six major HLAs (HLA-A, HLA-B, HLA-C, HLA-DR, HLADP, and HLA-DQ). Each HLA antigen has multiple isoforms in the human population, and each individual can have two different isoforms for each HLA due to the diploid nature of our genome. Therefore, a complete match would match twelve out of twelve isoforms. A cell or tissue donated from the same individual as, or an identical twin of, the intended recipient would have a perfect HLA-type and is referred to as syngeneic or autologous. It is also understood that certain factors including but not limited to ethnic background and race correlate with certain HLA-types.

Many major and minor HLA isoforms exist and it is understood that a suitable match can include a match between a subset of the major HLAs, all the major HLAs, some or all major and minor HLAs or any combination known to the art that mitigates immuno-rejection or GVHD. It is also understood that specific guidelines for what constitutes a good HLA-type match depends on many factors. Therefore, judgment must be made by one skilled in the art to assess the suitability of a given cell or tissue sample for transplant into a given individual.

HLA type can be determined using methods known in the art, including for example low resolution methods, such as by sero-typing, or using antibody based methods. Sero-typing is based on antibody recognition of HLA types. Sero-typing can distinguish between 28 different HLA-A genes, 59 HLA-B genes and 21 HLA-C genes. A perfect match by sero-typing methods would be a six out of six match referring to the two alleles for each HLA (A, B, and C) present in each individual. In certain instances, a five out of six match or less can be considered a good match as determined by one skilled in the art.

Other low or medium resolution methods to determine HLA type examine the HLA isoforms of the individual, but do not rely on determining the actual sequence of an individual's HLA alleles. Often, the donor is related to the individual receiving the sample and in such instances, sero-typing alone or in combination with other low or medium resolution methods can be sufficient to determine if a sample is suitable for administration. In instances where a donor is not related to the recipient, HLA type can be a five out of six or lower match. In such instances it can be useful to use cells or tissues with a lower match rather than expend time and effort to find a better HLA type match.

High resolution methods involve examining the specific sequence of the HLA genes or gene expression products (protein or RNA). High resolution methods can distinguish between thousands of different isoforms. HLA typing of the HSPCs and enhanced-HSPCs can be performed for six HLA loci, HLA-A, -B, and -DR, for example, at low resolution/split antigen level.

DNA-based testing methods can be utilized for HLA-DR typing. DNA-based testing can be used for HLA-A and -B. Transplant center guidelines for typing of subject, family and to confirm the HLA types of potential unrelated donors include, typing HLA-A, B, and -DR loci using primarily DNA-based testing methods at allele level resolution for DRBI and low resolution/split antigen level for HLA-A and -B. The typing of a subject and the selected donor can be performed using the same set of reagents, methodology, and interpretation criteria with fresh tissue samples to ensure HLA identity. Quality assurance and quality control for HLA testing are complied with.

Accordingly, compositions used in the methods described herein can include haplotyped enhanced-HSPCs. The enhanced-HSPCs can be HLA typed based on HLA-A, HLA-B, HLA-C, and HLA-DRB1. The enhanced-HSPCs can be HLA typed based on a matched HLA group with a specific human subject. The HLA haplotyped enhanced-HSPCs can include 6 out of 6 HLA matches with a specific human subject. The HLA haplotyped enhanced-HSPCs can include 5 out of 6 HLA matches with a specific human subject. The HLA haplotyped enhanced-HSPCs can include 4 out of 6 HLA matches with a specific human subject. HLA matching can be based on alleles or antigens, and combinations thereof.

### Percentages of Cells by Type in Population of Cells

The methods and compositions described herein can include a plurality of cells (e.g., a cell population) that is about 0.1% to about 1%, about 1% to about 3%, about 3% to about 5%, about 10% to about 15%, about 15%-20%, about 20%-25%, about 25%-30%, about 30%-35%, about 35%-40%, about 40%-45%, about 45%-50%, about 60%-70%, about 70%-80%, about 80%-90%, about 90%-95%, or about 95% to about 100% HSPCs. The population of cells can be about 0.1% to about 1% HSPCs. The population of cells can be about 1% to about 3% HSPCs. The population of cells can be about 3% to about 5% HSPCs. The population of cells can be about 10% to about 15% HSPCs. The population of cells can be about 15%-20% HSPCs. The population of cells can be about 20%-25% HSPCs. The population of cells can be about 25%-30% HSPCs. The population of cells can be about 30%-35% HSPCs. The population of cells can be about 35%-40% HSPCs. The population of cells can be about 40%-45% HSPCs. The population of cells can be about 45%-50% HSPCs. The population of cells can be about 60%-70% HSPCs. The population of cells can be about 70%-80% HSPCs. The population of cells can be about 80%-90% HSPCs. The population of cells can be about 90%-95% HSPCs. The population of cells can be about 95% to about 100% HSPCs.

The methods and compositions described herein can include a plurality of hematopoietic cells (e.g., a cell population) that is about 1%, 5%, 10%, 20%, 30%, 40%, 50%, 60%, 70%, 80%, 85%, 90%, 95%, 96%, 97%, 98%, 99%, or 100% HSPCs. The population of cells can be about 1% HSPCs. The population of cells can be about 5% HSPCs. The population of cells can be about 10% HSPCs. The population of cells can be about 20% HSPCs. The population of cells can be about 30% HSPCs. The population of cells can be about 40% HSPCs. The population of cells can be about 50% HSPCs. The population of cells can be about 60% HSPCs. The population of cells can be about 70% HSPCs. The population of cells can be about 80% HSPCs. The population of cells can be about 85% HSPCs. The population of cells can be about 90% HSPCs. The population of cells can be about 95% HSPCs. The population of cells can be about 96% HSPCs. The population of cells can be about 97% HSPCs. The population of cells can be about 98% HSPCs. The population of cells can be about 99% HSPCs. The population of cells can be about 100% HSPCs.

The populations of cells described above can include about 0.1% to about 1%, about 1% to about 3%, about 3% to about 5%, about 10%-about 15%, about 15%-20%, about 20%-25%, about 25%-30%, about 30%-35%, about 35%-40%, about 40%- 45%, about 45%-50%, about 60%-70%, about 70%-80%, about 80%-90%, about 90%-95%, or about 95% to about 100% CD34+ cells. The populations of cells described above can include about 1% CD34+ cells. The populations of cells described above can include about 3% CD34+ cells. The populations of cells described above can include about 5% CD34+ cells. The populations of cells described above can include about 10% CD34+ cells. The populations of cells described above can include about 20% CD34+ cells. The populations of cells described above can include about 30% CD34+ cells. The populations of cells described above can include about 40% CD34+ cells. The populations of cells described above can include about 50% CD34+ cells. The populations of cells described above can include about 60% CD34+ cells. The populations of cells described above can include about 70% CD34+ cells. The populations of cells described above can include about 80% CD34+ cells. The populations of cells described above can include about 85% CD34+ cells. The populations of cells described above can include about 90% CD34+ cells. The populations of cells described above can include about 95% CD34+ cells. The populations of cells described above can include about 96% CD34+ cells. The populations of cells described above can include about 97% CD34+ cells. The populations of cells described above can include about 98% CD34+ cells. The populations of cells described above can include about 99% CD34+ cells. The populations of cells described above can include about 100% CD34+ cells.

Cells can undergo a number of manipulations prior to being modulated. For example, cells can be purified, expanded, split, cryopreserved, or enzymatically digested, prior to or after modulation with one or more agents to increase therapeutic potential and/or expand the cell population.

In one embodiment, the hematopoietic cells may be enriched or purified by depletion of other cell types, or purification for a desired cell marker. Hematopoietic cells described herein for use in the methods described herein can be depleted of mature hematopoietic cells such as T cells, B cells, NK cells, dendritic cells, monocytes, granulocytes, erythroid cells, and their committed precursors from bone marrow aspirate, umbilical cord blood, or mobilized peripheral blood (mobilized leukapheresis product). Mature, lineage committed cells can be depleted by immunodepletion using methods known in the art. For example, immunodepletion can be performed by labeling solid substrates with antibodies that bind to a panel of lineage antigens: (e.g., CD2, CD3, CD11b, CD14, CD15, CD16, CD19, CD56, CD123, or CD235a). A subsequent step can be performed to further purify the population of cells. The subsequent step includes a substrate labeled with antibodies that binds to the CD34+ antigen. Such techniques can be used to isolate primitive hematopoietic stem and progenitor cells. Kits are commercially available for purifying hematopoietic stem and progenitor cells from various cell sources and in particular embodiments, these kits are suitable for use with the methods of the present invention. Exemplary commercially available kits for purifying hematopoietic stem and progenitor cells include, but are not limited to Lineage (Lin) Depletion Kit (Miltenyi Biotec); CD34+ enrichment kit (Miltenyi Biotec); RosettaSep (Stem Cell Technologies).

The plurality of hematopoietic cells (e.g., "cell population" or "population") can include less than about 30%, 25%, 20%, 15%, 10% or 5% mesenchymal stem cells. The plurality of hematopoietic cells can include no more than about 10% mesenchymal stem cells. Mesenchymal stem cells (MSCs) are multipotent stem cells that can differentiate readily into lineages including osteoblasts, myocytes, chondrocytes, and adipocytes (Pittenger, et al., Science, Vol. 284, pg. 143 (1999); Haynesworth, et al., Bone, Vol. 13, pg. 69 (1992); Prockop, Science, Vol. 276, pg. 71 (1997)).

The plurality of hematopoietic cells can include less than about 30%, 25%, 20%, 15%, 10% or 5% endothelial progenitor cells. The plurality of hematopoietic cells can include less than about 10% endothelial progenitor cells. As used herein, the term "endothelial progenitor cell" refers to a multi potent or unipotent cell with the potential to differentiate into vascular endothelial cells. The plurality of hematopoietic cells can include no more than about 10% mesenchymal stem cells or endothelial progenitor cells.

The plurality of hematopoietic cells as obtained from a donor (*e.g*., isolated from a donor), or as otherwise provided, can be substantially free of mesenchymal stem cells and/or endothelial progenitor cells (*e.g*., less than about 10% mesenchymal stem cells and less than about 10% endothelial progenitor cells). The plurality of hematopoietic cells can alternatively be depleted of mesenchymal stem cells and/or endothelial progenitor cells using methods known in the art, for example, using immunomagnetic selection techniques, fluorescence activated cell sorting, or a combination therein. The depletion methods can further include using at least one antibody specific for at least one of the cell-surface markers described herein.

The plurality of hematopoietic cells can be depleted of endothelial progenitor cells, including endothelial progenitor cells positive for the CD14 cell surface marker and negative for CD45 (CD14+/CD45-) and/or cells positive for VWF (Von willebrand Factor) (VWF +). The plurality of HSPCs can be depleted of cells positive for CD73 and/or CD140B cell surface markers (CD73+/CD140B+). The plurality of hematopoietic cells can include cells positive for the cell surface marker CD34, and include less than about 30%, 25%, 20%, 15%, 10% or 5% of cells positive for a cell surface marker selected from the group consisting of CD73, CD140B, CD14 and VWF.

The plurality of hematopoietic cells can include CD34+ cells and include less than about 30%, 25%, 20%, 15%, 10% or 5% CD14+/CD45- cells. The plurality of hematopoietic cells can include CD34+ cells and include less than about 30%, 25%, 20%, 15%, 10% or 5% VWF+ cells. The plurality of HSPCs can include CD34+ cells and include less than about 30%, 25%, 20%, 15%, 10% or 5% CD140B+ cells.

The plurality of human hematopoietic cells can include CD34+ hematopoietic cells and include less than about 30%, 25%, 20%, 15%, 10% or 5% of CD 14+/CD45- cells, VWF+ cells, CD73+ cells, and CD140B+ cells. The plurality of human hematopoietic cells can be positive for the cell surface marker CD34 and negative for at least one cell surface marker from the group consisting of CD14, VWF, CD73, and CD140B. The plurality of human hematopoietic cells can be positive for the cell surface marker CD34 and negative for the cell surface markers CD14, VWF, CD73, and CD140B.

The hematopoietic cells described herein may or may not be expanded or split prior to administration. The hematopoietic cells may or may not be expanded ex *vivo* or *in vitro* prior to administration to a subject. An unexpanded plurality of modulated hematopoietic cells can be obtained and administered to a subject, where the plurality of modulated hematopoietic cells are derived using the methods described herein (*e.g*., treating hematopoietic cells ex *vivo* as described herein). The methods to generate a plurality of modulated hematopoietic cells can further include a washing step to remove the exogenous agent prior to administration. The cells can be obtained from a donor (*e.g*., via cord blood) and not expanded prior to or after ex *vivo* treatment of the cells as described herein, or at any time prior to administration to a subject. Thus, an unexpanded plurality of hematopoietic cells can be *ex vivo* contacted as described herein, thereby generating a plurality of modulated hematopoietic cells as described herein, and administered to a subject prior to any substantial *ex vivo* cell division of the cells in the population, or prior to the time required for any substantial cell division *ex vivo.* An unexpanded population of cells can be *ex vivo* contacted as described herein and administered to a subject prior to any substantial *ex vivo* mitosis of the cells in the population, or prior to the time required for any substantial mitosis *ex vivo.* An unexpanded plurality of hematopoietic cells can be *ex vivo* treated as described herein and administered to a subject prior to the doubling time of the cells in the population. An unexpanded plurality of hematopoietic cells can be ex *vivo* treated as described herein and administered to a subject within 1, 2, 3, 4, 5, 6, 7, 8, 9, 10, 11, 12, or 24 hours of treatment of the cells using the methods described herein, as well as any time intervening these specific time points. An unexpanded plurality of hematopoietic cells can be *ex vivo* contacted as described herein, and administered to a subject within about 2 hours of treatment of the cells. Further, an unexpanded plurality of hematopoietic cells can be *ex vivo* contacted as described herein, and subsequently cryopreserved for a period of time prior to administration to a subject. Cryopreservation of the modulated hematopoietic cells may occur within 1, 2, 3, 4, 5, 6, 7, 8, 9, 10, 11, 12, or 24 hours of treatment of the cells using the methods described herein, as well as any time intervening these specific time points.

Hematopoietic cells may or may not be cultured prior to *ex vivo* contact with an exogenous agent (*e.g*., a glucocorticoid) as described herein. In aspects where the hematopoietic cells are cultured prior to *ex vivo* contact with an exogenous agent, the hematopoietic cells may be cultured less than about 24 hours, less than about 12 hours, less than about 10 hours, less than about 8 hours, less than about 6 hours, less than about 4 hours, or less than about two hours. Hematopoietic cells may or may not be cultured prior to administration to a subject. Where the hematopoietic cells are cultured prior to administration to a subject, the hematopoietic cells can be cultured for less than about 24 hours, or less than about 12 hours, less than about 10 hours, less than about 8 hours, less than about 6 hours, less than about 4 hours, or less than about two hours. The hematopoietic cells can be cultured for about 2 hours. The hematopoietic cells can be cultured for less than about 24 hours. The hematopoietic cells can be cultured for less than about 12 hours, 10 hours, 8 hours, 6 hours, 4 hours, or two hours. The hematopoietic cells can be cultured for about 2 hours.

The hematopoietic cells may or may not be expanded prior to contact of the hematopoietic cells with exogenous agents to obtain modulated hematopoietic cells as described herein. Hematopoietic cells, whether obtained from cord blood, bone marrow, peripheral blood, Wharton's jelly, placental blood or other source, can be grown or expanded in any suitable, commercially available or custom defined medium, with or without serum, as desired (see, *e.g*., Hartshorn et al., Cell Technology for Cell Products, pages 221-224, R. Smith, Editor; Springer Netherlands, 2007, herein incorporated by reference in its entirety). For instance, serum free medium can utilize albumin and/or transferrin, which have been shown to be useful for the growth and expansion of CD34+ cells in serum free medium.

The hematopoietic cells that are *ex vivo* contacted with an agent as described herein and subsequently administered to a subject can be a heterogeneous population of cells (*e.g*. a plurality of heterogeneous hematopoietic cells). The plurality of heterogeneous hematopoietic cells can include whole bone marrow, umbilical cord blood, mobilized peripheral blood, hematopoietic stem cells, hematopoietic progenitor cells, and the progeny of hematopoietic stem and progenitor cells, including, for example, granulocytes (*e*.*g*., pro-myelocytes, myelocytes, metamyelocytes, neutrophils, eosinophils, basophils), erythrocytes (*e*.*g*., reticulocytes, erythrocytes), thrombocytes (*e.g*., megakaryoblasts, platelet producing megakaryocytes, platelets), and monocytes (*e*.*g*., monocytes, macrophages).

Contacting human cord blood, bone marrow cells, or mobilized peripheral blood cells having hematopoietic cells with an exogenous agent described herein that increases PD-L1 and/or IDO-1, can increase the *in vivo* immuno-regulatory properties of HSPC administered to a subject. Contacting human cord blood, bone marrow cells, or mobilized peripheral blood cells having hematopoietic cells with an exogenous agent described herein at a temperature greater than about room temperature can increase the *in vivo* immuno-regulatory properties of the HSPC population administered to a subject. Contacting human cord blood, bone marrow cells, or mobilized peripheral blood cells having hematopoietic cells with an exogenous agent described herein for about 120 minutes at a temperature greater than about room temperature can increase the *in vivo* immuno-regulatory properties of the hematopoietic cell population administered to a subject.

Contacting a purified population of Lin(-)CD34+ HSPCs with an exogenous agent described herein can increase the *in vivo* expansion of the hematopoietic stem or progenitor cell population administered to a subject. Contacting a purified population of Lin(-)CD34+ HSPCs with an exogenous agent described herein at a temperature greater than about room temperature can increase the immuno-regulatory properties of the hematopoietic stem or progenitor cell population administered to a subject. Contacting a purified population of Lin(-)CD34+ HSPCs with an exogenous agent described herein for about 120 minutes at a temperature greater than about room temperature can increase immuno-regulatory properties of the hematopoietic stem or progenitor cell population administered to a subject.

In various embodiments, the cells are not genetically modified cells. In other embodiments, the cells are genetically modified with a polynucleotide, such as, for example a retroviral or lentiviral vector comprising a protein coding gene sequence. In some embodiments, the cell is genetically modified to correct a genetic defect and in other embodiments, the cell is genetically modified to increase or decrease production of a wild-type or mutant protein. Polynucleotides used to increase expression of a protein in a cell may comprise polynucleotide sequences to direct appropriate expression in the cell and a polynucleotide encoding the polypeptide sequence. Polynucleotides used to decrease expression of a protein in a cell may comprise polynucleotide sequences that target polynucleotides encoding the wild type polypeptide sequence for degradation. In some embodiments, cells modulated according to the invention are purified cells or a population of cells comprising a mixture of cell types. In some embodiments of the invention, hematopoietic stem and progenitor cells (HSPCs) are modulated to increase PD-L1 and/or IDO-1 expression. Hematopoietic stem cells are multipotent stem cells that give rise to all the blood cell types of an organism, including myeloid (e.g., monocytes and macrophages, neutrophils, basophils, eosinophils, erythrocytes, megakaryocytes/platelets, dendritic cells), and lymphoid lineages (e.g., T-cells, B-cells, NK-cells), and others known in the art (See Fei, R., et al., U.S. Patent No. 5,635,387; McGlave, et al., U.S. Patent No. 5,460,964; Simmons, P., et al., U.S. Patent No. 5,677,136; Tsukamoto, et al., U.S. Patent No. 5,750,397; Schwartz, et al., U.S. Patent No. 5,759,793; Tsukamoto, et al., U.S. Patent No. 5,716,827). Hematopoietic progenitor cells give rise to committed hematopoietic progenitor cells that are capable of generating the entire repertoire of mature blood cells over the lifetime of an organism. In some aspects of the invention, CD34+ HSPCs are modulated to achieve increased PD-L1 and/or IDO-1 expression.

### III. METHODS OF USE

Modulated cells can be useful in a variety of clinical settings, including cell transplantation, the treatment of disorders and diseases, and gene therapy. Accordingly, the phrase "a subject in need thereof' refers to an individual that is to be treated for a disease or disorder as disclosed in this specification. In particular embodiments, modulated cells find use in treating immunological or inflammatory disorders. In some aspects, modulated cells treat immunological or inflammatory disorders by inhibiting an immune response in a subject. As used herein, the phrase "immunological disorder" includes, but is not limited to, autoimmune disorders, graft-versus host disease, and transplant rejection.

The modulated cells of the invention find use in treating any autoimmune disorder that responds to the administration of a cell having increased PD-L1 and/or IDO-1 expression. Examples of autoimmune disorders that may be treated with the modulated cells include, but are not limited to, acute myocardial infarction, ischemic stroke, type 1 diabetes, diabetes mellitus, multiple sclerosis, acute disseminated encephalomyelitis, inflammatory demyelinating diseases, lupus, Crohn's disease, osteoarthritis, rheumatoid arthritis, psoriatic arthritis, ulcerative colitis, dermatitis, irritable bowel syndrome, vitiligo, Graves' disease, Hashimoto's disease, Addison's disease, polymyositis, dermatomyositis, myasthenia gravis, autoimmune hepatitis, Sjögren's syndrome, autoimmune gastritis, sclerosis, psoriasis, asthma, or Wegener's granulomatosis.

In one embodiment, the modulated cells treat inflammatory conditions or disorders. As used herein, an inflammatory condition or disorder is a condition or disorder which has a basis in or component of inflammation. It is recognized that some immunological disorders have an inflammatory basis or component, and thus they are also categorized as inflammatory conditions, as discussed further below. Examples of inflammatory conditions which may be treated by the administration of the modulated cells of the invention include, but are not limited to, inflammation of the lungs, joints, connective tissue, eyes, nose, bowel, kidney, liver, skin, central nervous system, endocrine system, vascular system and heart. In certain embodiments, inflammatory conditions which may be treated by the current invention include inflammation due to the infiltration of leukocytes or other immune effector cells into affected tissue. Other relevant examples of inflammatory conditions which may be treated by the present invention include inflammation caused by infectious agents, including, but not limited to, viruses, bacteria fungi and parasites.

Inflammatory lung conditions include, but are not limited to, asthma, adult respiratory distress syndrome, bronchitis, pulmonary inflammation, pulmonary fibrosis, and cystic fibrosis (which may additionally or alternatively involve the gastro-intestinal tract or other tissue(s)). Inflammatory joint conditions include rheumatoid arthritis, rheumatoid spondylitis, juvenile rheumatoid arthritis, osteoarthritis, gouty arthritis and other arthritic conditions. Eye diseases with an inflammatory component include, but are not limited to, uveitis (including iritis), conjunctivitis, scleritis, keratoconjunctivitis sicca, and retinal diseases, including, but not limited to, diabetic retinopathy, retinopathy of prematurity, retinitis pigmentosa, and dry and wet age-related macular degeneration. Inflammatory bowel conditions include Crohn's disease, ulcerative colitis and distal proctitis.

Inflammatory skin diseases include, but are not limited to, conditions associated with cell proliferation, such as psoriasis, eczema and dermatitis, (e.g., eczematous dermatitides, topic and seborrheic dermatitis, allergic or irritant contact dermatitis, eczema craquelee, photoallergic dermatitis, phototoxic dermatitis, phytophotodermatitis, radiation dermatitis, and stasis dermatitis). Other inflammatory skin diseases include, but are not limited to, scleroderma, ulcers and erosions resulting from trauma, burns, bullous disorders, or ischemia of the skin or mucous membranes, several forms of ichthyoses, epidermolysis bullosae, hypertrophic scars, keloids, cutaneous changes of intrinsic aging, photoaging, frictional blistering caused by mechanical shearing of the skin and cutaneous atrophy resulting from the topical use of corticosteroids. Additional inflammatory skin conditions include inflammation of mucous membranes, such as cheilitis, chapped lips, nasal irritation, mucositis and vulvovaginitis.

Inflammatory disorders of the endocrine system include, but are not limited to, autoimmune thyroiditis (Hashimoto's disease), Type I diabetes, Type II diabetes, and acute and chronic inflammation of the adrenal cortex. Inflammatory conditions of the cardiovascular system include, but are not limited to, coronary infarct damage, peripheral vascular disease, myocarditis, vasculitis, revascularization of stenosis, atherosclerosis, and vascular disease associated with Type II diabetes.

Inflammatory conditions of the kidney include, but are not limited to, glomerulonephritis, interstitial nephritis, lupus nephritis, nephritis secondary to Wegener's disease, acute renal failure secondary to acute nephritis, Goodpasture's syndrome, post-obstructive syndrome and tubular ischemia.

Inflammatory conditions of the liver include, but are not limited to, hepatitis (arising from viral infection, autoimmune responses, drug treatments, toxins, environmental agents, or as a secondary consequence of a primary disorder), biliary atresia, primary biliary cirrhosis and primary sclerosing cholangitis.

Inflammatory conditions of the central nervous system include, but are not limited to, multiple sclerosis and neurodegenerative diseases such as Alzheimer's disease, Parkinson's disease, or dementia associated with HIV infection.

Other inflammatory conditions include periodontal disease, tissue necrosis in chronic inflammation, endotoxin shock, smooth muscle proliferation disorders, graft versus host disease, tissue damage following ischemia reperfusion injury, and tissue rejection following transplant surgery.

The present invention further provides a method of treating inflammation associated with wound healing, including post-surgical wound healing in a patient comprising administering to said patient a composition comprising the modulated cells of the invention.

It should be noted that the modulated cells of the current invention may be used to treat any disease which has an inflammatory component, such as those diseases cited above. Further, the inflammatory conditions cited above are meant to be exemplary rather than exhaustive.

Those skilled in the art would recognize that additional inflammatory conditions (e.g., systemic or local immune imbalance or dysfunction due to an injury, an insult, infection, inherited disorder, or an environmental intoxicant or perturbant to the subject's physiology) may be treated the modulated cells of the current invention. Thus, the methods of the current invention may be used to treat any disease which has an inflammatory component, including, but not limited to, those diseases cited above.

In one embodiment, the patient receiving the modulated cells is not expected to have or need a transplant. In one aspect, the patient receiving the modulated cells has not received a transplant. In one aspect, the patient receiving the modulated cells is not a candidate for hematopoietic transplant to reconstitute the hematopoietic compartment. In one non-limiting embodiment, modulated cells are administered to a subject that has received no conditioning prior to administration of the cells. "Conditioning" as used herein is meant to convey treatment typically administered prior to hematopoietic stem cell transplantation. Typically such conditioning is performed with radio- or chemo-therapies. In one aspect, the modulated cells are administered to a subject who has not had high-dose (myeloablative) conditioning. In another aspect, the modulated cells are administered to a patient who has not had either high-dose or reduced-intensity conditioning. In yet another aspect, the modulated cells are administered to a patient who has not had any of high-dose, reduced-intensity, or nonmyeloablative conditioning.

In another embodiment, the modulated cells also find use in treating immunological disorders that arise from transplantation therapy, such as graft-versus-host disease and transplant rejection, for example. Thus, modulated cells may be used to treat graft versus host disease or transplant rejection that results from a bone marrow transplant, solid organ transplant, or cell therapy (e.g. any composition comprising isolated stem cells). Transplants in the context of cell therapy, include, but are not limited to, the administration of genetically engineered cells that have been modified to produce a desired protein or polynucleotide in a subject. In aspects of the invention, modulated cells are administered to the subject before or at the time of transplantation so as to prevent graft-versus- host disease and/or transplant rejection. Without being limited to any particular theory or mechanism, treating a subject with modulated cells prior to or at the time of transplantation dampens the immunological response in the transplant recipient and improves transplant acceptance and engraftment.

In one non-limiting embodiment, modulated cells are administered to a subject that has received a bone marrow transplant (or cell therapy), wherein the transplant is directed to hematopoietic engraftment or reconstitution. Such subjects include, but are not limited to, subjects undergoing chemotherapy or radiation therapy for cancer, as well as subjects suffering from (e.g., afflicted with) non-malignant blood disorders, particularly immunodeficiencies (e.g. SCID, Fanconi's anemia, severe aplastic anemia, or congenital hemoglobinopathies, or metabolic storage diseases, such as Hurler's disease, Hunter's disease, mannosidosis, among others) or cancer, particularly hematological malignancies, such as acute leukemia, chronic leukemia (myeloid or lymphoid), lymphoma (Hodgkin's or non-Hodgkin's), multiple myeloma, myelodysplastic syndrome, or non-hematological cancers such as solid tumors (including breast cancer, ovarian cancer, brain cancer, prostate cancer, lung cancer, colon cancer, skin cancer, liver cancer, or pancreatic cancer). In some embodiments, the modulated cells are HSCs, or a modulated bone marrow transplant, are administered for the purpose of providing hematopoietic engraftment or reconstitution in a subject, wherein the modulated cells or bone marrow avoid, or are made less susceptible to, graft-versus-host disease or transplant rejection.

Subjects also include individuals that have received a bone marrow transplant or cell therapy in the treatment of aplastic anemia, myelodysplasia, thalassemaia, sickle-cell disease or Wiskott- Aldrich syndrome. In some embodiments, the subject suffers from a disorder that is the result of an undesired side effect or complication of another primary treatment, such as radiation therapy, chemotherapy, or treatment with a bone marrow suppressive drug, such as zidovadine, chloramphenical or gangciclovir. Such disorders include neutropenias, anemias, thrombocytopenia, and immune dysfunction.

Administration of an "amount" of modulated cells to a subject refers to administration of "an amount effective," to achieve the desired therapeutic or prophylactic result, including without limitation, treatment of the subject. A "therapeutically effective amount" of cells for purposes herein is thus determined by such considerations as are known in the art, and may vary according to factors such as the disease state, age, sex, and weight of the individual, and the ability of the cells to elicit a desired response in the individual. The term "therapeutically effective amount" includes an amount that is effective to "treat" a subject (e.g., a patient). A therapeutically effective amount is also one in which any toxic or detrimental effects of the cells are outweighed by the therapeutically beneficial effects.

A "prophylactically effective amount" refers to an amount of cells having therapeutic potential that is effective to achieve the desired prophylactic result. Typically but not necessarily, since a prophylactic dose is used in subjects prior to or at an earlier stage of disease, the prophylactically effective amount is less than the therapeutically effective amount.

The amount of modulated hematopoietic cells administered to a subject can be, for example, the amount of HSPC in a partial or single cord of blood. The amount of modulated hematopoietic cells can be at least 2×10⁶ cells, at least 2.5×10⁶ cells, at least 3×10⁶ cells, at least 4×10⁶ cells, at least 5×10⁶ cells, at least 1×10⁷ cells, at least 1.5×10⁷ cells, at least 2×10⁷ cells, at least 2.5×10⁷ cells, or at least 3×10⁷ cells.

The amount of modulated hematopoietic cells administered to a subject can be about 2 × 10⁶ cells, about 3×10⁶ cells, about 5×10⁶ cells, about 7×10⁶ cells, about 10×10⁶ cells, about 15×10⁶ cells, about 1.7×10⁷ cells, about 2.0×10⁷ cells about 2.5×10⁷ cells, about 3.0×10⁷ cells, about 5.0×10⁷ cells, about 1.0×10⁸ cells, about 3.0×10⁸ cells, about 5.0×10⁸ cells, about 1.0×10⁹ cells, about 3.0×10⁹ cells, about 5.0×10⁹ cells, or about 1.0×10¹⁰ cells.

The amount of modulated hematopoietic cells administered to the subject can be, for example, about 2 ×10⁶ cells to about 2×10¹⁰ cells; about 2×10⁶ cells to about 7×10⁹ cells; about 2×10⁶ cells to about 5×10⁷ cells; about 2×10⁶ cells to about 3×10⁷ cells; or about 2×10⁶ cells to about 2.5×10⁷ cells. The amount of modulated hematopoietic cells administered to the subject can, for example, be about 2×10⁶ cells. The amount of modulated hematopoietic cells administered to the subject can, for example, be about 5×10⁶ cells. The amount of modulated hematopoietic cells administered to the subject can, for example, be about 1×10⁷ cells. The amount of modulated hematopoietic cells administered to the subject can, for example, be about 5×10⁷ cells. The modulated hematopoietic cells can be administered to the subject in an amount less than about 2×10⁸ cells. The modulated hematopoietic cells can be administered to the subject in an amount less than about 2×10⁷ cells.

The amount of modulated hematopoietic cells administered to the subject can be at least about 1×10⁶ cells/kg of bodyweight, at least about 1.25×10⁶ cells/kg of bodyweight, at least about 1.5×10⁶ cells/kg of bodyweight, at least about 1.75×10⁶ cells/kg of bodyweight, at least about 2×10⁶ cells/kg of bodyweight, at least about 2.5×10⁶ cells/kg of bodyweight, at least about 3×10⁶ cells/kg of bodyweight, at least about 4×10⁶ cells/kg of bodyweight, at least about 5×10⁶ cells/kg of bodyweight, at least about 1×10⁷ cells/kg of bodyweight, at least about 1.5×10⁷ cells/kg of bodyweight, at least about 2×10⁷ cells/kg of bodyweight, at least about 2.5×10⁷ cells/kg of bodyweight, at least about 3×10⁷ cells/kg of bodyweight, at least about 5×10⁷ cells/kg of bodyweight, at least about 7×10⁷ cells/kg of bodyweight, at least about 1×10⁸ cells/kg of bodyweight, at least about 3×10⁸ cells/kg of bodyweight, at least about 5×10⁸ cells/kg of bodyweight, at least about 7×10⁸ cells/kg of bodyweight, at least about 1×10⁹ cells/kg of bodyweight, at least about 3×10⁹ cells/kg of bodyweight, at least about 5×10⁹ cells/kg of bodyweight, at least about 7×10⁹ cells/kg of bodyweight, or at least about 3×10¹⁰ cells/kg of bodyweight.

In another embodiment, the amount of modulated hematopoietic cells administered to a subject is the amount of hematopoietic cells in a partial or single cord of blood, or about 1×10⁵ cells to about 2×10⁸ cells; about 1.0×10⁶ cells to about 2×10⁷ cells; about 2×10⁶ cells to about 1×10⁷ cells, about 2×10⁶ cells to about 7×10⁶ cells, about 2×10⁶ cells to about 5×10⁶ cells, or about 2×10⁶ cells to about 3×10⁶ cells.

The modulated cells of the present invention may be administered to a subject at one or more times. For instance, the present methods contemplate one, 2, 3, 4, 5, 6, 7, 8, 9, 10, or more administrations. In one embodiment, repeated administrations may persist until one or more symptoms of an autoimmune disorder are decreased. In another embodiment, repeated administrations may persist for the lifetime of the subject to maintain a certain level of symptoms.

In one embodiment, the administrations may be an interval ranging from twice every week to once every six months. For instance, the administrations may be every two weeks, every three weeks, every four weeks, monthly, every other month, every three months, every four months, or every six months. In one embodiment, the interval between administrations of modulated cells is every two weeks. In one embodiment the interval between administrations of modulated cells is every six weeks.

In one embodiment, the patient may be administered an initial therapy cycle, or prescribed therapy regimen, with an initial dose at an initial interval and thereafter be administered another therapy cycle with a maintenance dose and maintenance interval. In one embodiment, the initial dose is a higher dose than the maintenance dose. Alternatively, the initial dose may be lower than the maintenance dose, as potential side-effects are observed. Further, in one embodiment the maintenance interval may be longer than the initial interval in the therapy cycle, such as with "booster" administrations. In another embodiment, the maintenance interval may be shorter than the initial interval.

Suitable methods for administering modulated cells used in the methods described herein include parenteral administration, including, but not limited to methods of intravascular administration, such as intravenous and intraarterial administration. Additional illustrative methods for administering cells of the invention include intramuscular, intrathecal, intracapsular, intraorbital, intracardiac, intradermal, intraperitoneal, transtracheal, subcutaneous (subdermal), subcuticular, intraarticular, subcapsular, subarachnoid, intraspinal and intrastemal injection and infusion.

In one embodiment, the modulated cells are administered locally. In another embodiment, the modulated cells are administered systemically.

In one embodiment, the route of administration is not a route used for hematopoietic transplant. For instance, hematopoietic transplant is generally systemic intravenous infusion. Thus, local administrations or non-intravenous administrations are not routes used for hematopoietic transplant.

Therapeutic advantages may be realized through combination regimens including the present compositions. Those skilled in the art will appreciate that the modulated cells described herein may be administered as part of a combination therapy approach to the treatment of an immunomodulatory disease or an inflammatory condition. For instance, the present modulated hematopoietic cells expressing increased levels of PD-L1 and/or IDO-1 may be combined with a second active agent. In combination therapy the respective agents may be administered simultaneously, or sequentially in any order. When administered sequentially, it may be preferred that the components be administered by the same route.

Alternatively, the components may be formulated together in a single dosage unit as a combination product. Suitable agents which may be used in combination with the compositions of the present invention will be known to those of ordinary skill in the art.

In certain embodiments, the present invention provides a kit comprising: a) one or more single dosage forms comprising a population of modulated hematopoietic cells of the invention; b) one or more single dosage forms of second active agent for use in a combination therapy and c) instructions for the administration of the population of modulated hematopoietic cells of the invention and the second active agent.

In one aspect, the present invention provides a kit comprising: a) a pharmaceutical formulation (e.g., one or more single dosage forms) comprising a population of modulated hematopoietic cells of the invention; and b) instructions for the administration of the pharmaceutical formulation e.g., for treating or preventing a disorder or condition as discussed above, e.g., inflammatory disease.

Particular embodiments of the present invention now will be described more fully by the following examples. This invention may, however, be embodied in many different forms and should not be construed as limited to the embodiments set forth herein; rather, these embodiments are provided so that this disclosure will be thorough and complete, and will fully convey the scope of the invention to those skilled in the art.

### EXAMPLE 1 - Elevated gene expression levels of PD-L1 (CD274) or IDO-1 in human stem and progenitor cells

Human CD34+ stem and progenitor cells isolated from mobilized peripheral blood from three donors were ex *vivo* treated in STEMSPAN® (StemCell Technologies) for 24 hours at 37°C with one or more exogenous agents. Following cell treatments, gross mRNA levels were normalized against gross mRNA levels from the untreated cells before RT-qPCR. Levels of PD-L1 or IDO-1 mRNA were quantified from PICOPURE® isolated mRNA (Life Technologies) using an Assay on Demand TAQMAN® RT-qPCR assay (Life Technologies).

Results of PD-L1 Expression After Modulation are shown in Table 1.

| **Table 1: PD-L1 Expression after Modulation With Agent** | | | |
|---|---|---|---|
| **Compound name** | **Class/MOA** | **%Viability** | **PD-L1 fold change** |
| Tyrphostin AG 835 | Protein tyrosine kinase inhibitor | 61.3 | 32.57 |
| Vigabatrin | GABA transaminase inhibitor | 67 | 16.76 |
| Betamethasone | Glucocorticoid | 86.8 | 4.16 |
| Fluocinolone acetonide | Glucocorticoid | 82.9 | 10.58 |
| Nitrofural | Antibacterial | 87.2 | 10.28 |
| Clobetasol propionate | Glucocorticoid | 83 | 9.27 |
| Clocortolone pivalate | Glucocorticoid | 84.9 | 8.64 |
| Fluphenazine | Dopamine receptor antagonist | 86 | 7.58 |
| Flumethasone | Glucocorticoid | 89.6 | 7.58 |

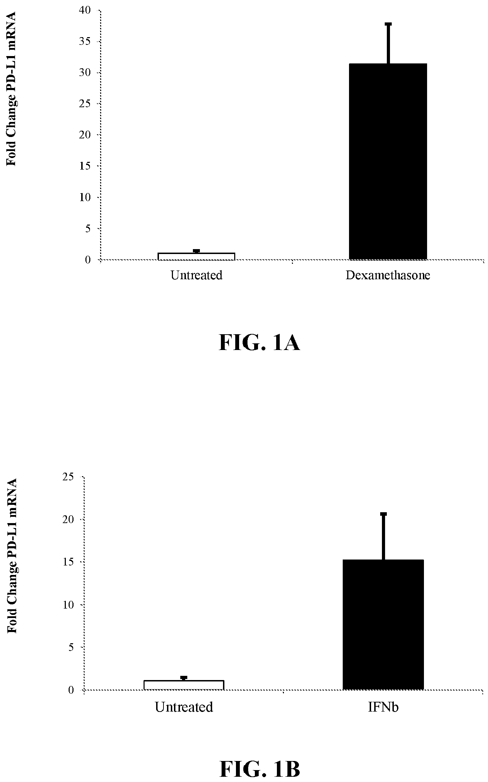

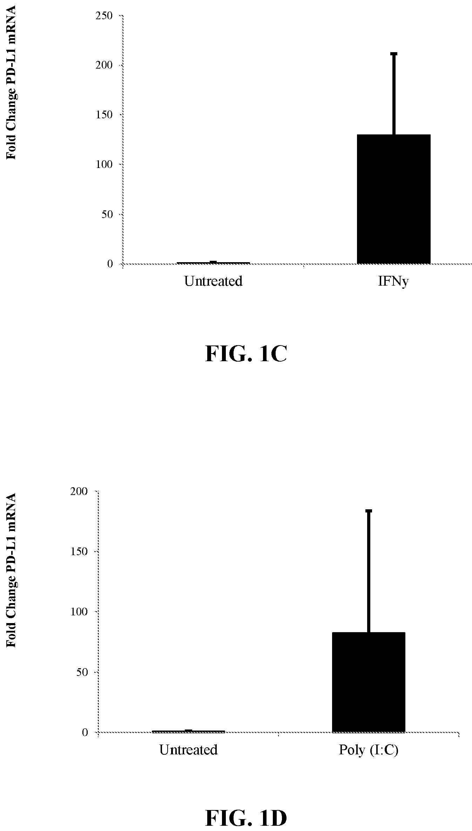

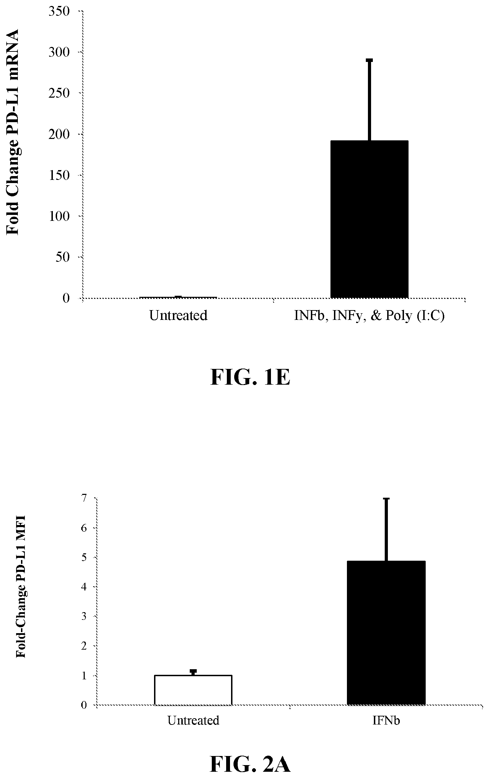

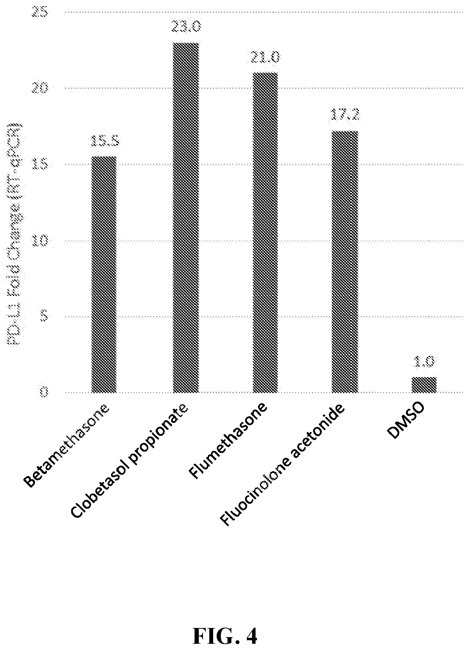

Fig. 1 shows results of PD-L1 gene expression levels as the mean of three individual donors of CD34+ cells treated with a single exogenous agent (A) 10µM Dexamethasone; (B) 1000U/mL Interferon beta (IFNβ); (C) 5ng/mL Interferon gamma (IFNγ); (D) 10µg/mL High Molecular Weight Polyinosinic- polycytidylic acid (Poly (I:C)) or multiple exogenous agents; (E) 1000U/mL IFNβ, 5ng/mL IFNγ, and 10µg/mL Poly (I:C). Fig. 4 shows results of modulation with additional glucocorticoids for 24 hours at 37°C.

Results of IDO-1 Expression after modulation are shown in Table 2.

| **Table 2: IDO-1 Expression After Modulation With Agent** | | | |
|---|---|---|---|
| **Exogenous Agent** | **Class/MOA** | **%Viability** | **IDO-1 fold change** |
| Gemcitabine | Antineoplastic | 81.4 | 30.13 |
| Fluphenazine | Dopamine receptor antagonist | 86 | 14.19 |
| Isometheptene mucate | Adrenergic receptor agonist | 83.7 | 6.98 |
| Dihydrostreptomycin sulfate | Ribosomal protein synthesis inhibitor | 84.5 | 6.19 |
| Protriptyline | Adrenergic reuptake inhibitor | 79.4 | 5.74 |
| Telenzepine | M1 muscarinic antagonist | 88.9 | 4.82 |
| Cyclobenzaprine | Serotonin receptor antagonist | 72.7 | 4.65 |
| Letrozole | Antineoplastic | 81.3 | 4.38 |
| Fludarabine | Antineoplastic | 82.5 | 4.22 |
| 4-aminosalicylic acid | NF-kB inhibitor | 85.3 | 4.07 |

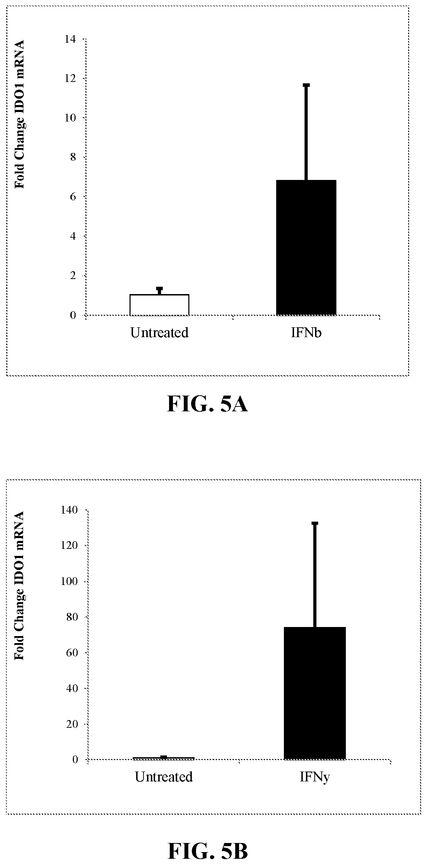

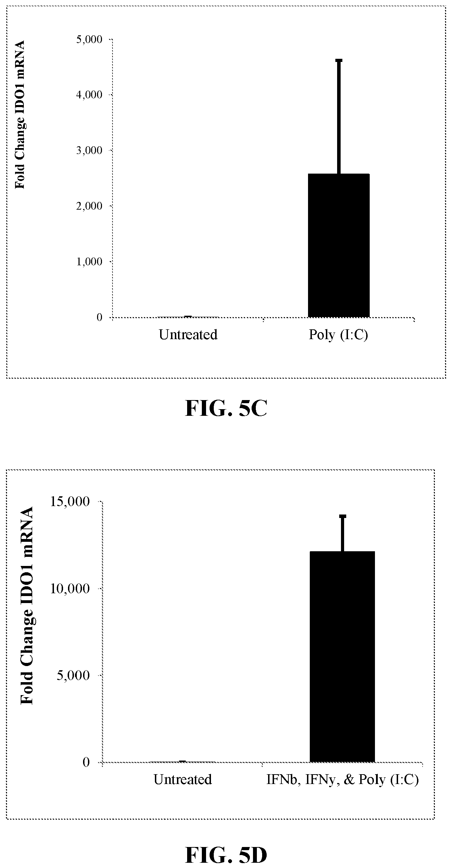

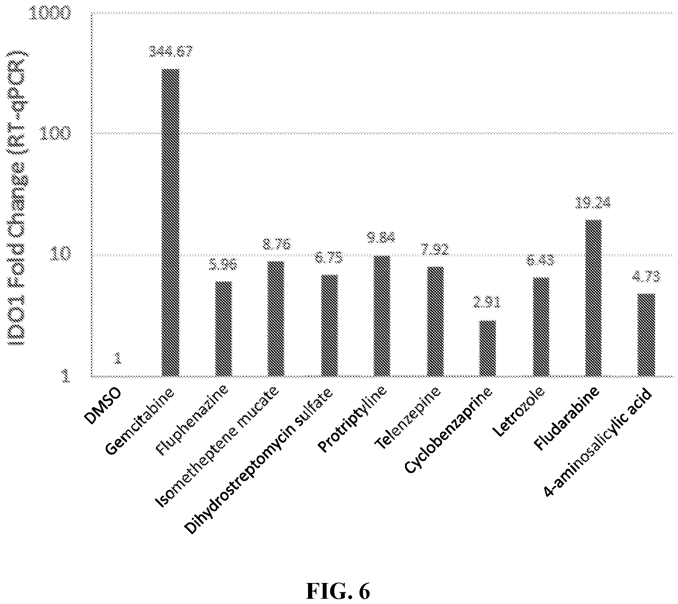

Figs. 5A-D show results of the IDO-1 gene expression levels, as the mean of three individual donors of CD34+ cells treated with a single exogenous agent (A) 1000U/mL Interferon beta (IFNβ); (B) 5ng/mL Interferon gamma (IFNγ); (C) 10µg/mL High Molecular Weight Polyinosinic- polycytidylic acid (Poly (I:C)) or multiple exogenous agents; (D) 1000U/mL IFNβ, 5ng/mL IFNγ, and 10µg/mL Poly (I:C). Fig. 6 shows incubation for 24 hours at 37° C with additional exogenous agents, including antineoplastic agents, dopamine receptor antagonists and others.

EXAMPLE 2 - Elevated levels of PD-L1 (CD274) or IDO-1 surface protein on human stem and progenitor cells.

Human CD34+ stem and progenitor cells (HSCs) isolated from mobilized peripheral blood were *ex vivo* treated in STEMSPAN® serum-free expansion medium (SFEM) (StemCell Technologies) with stem cell factor (SCF), Flt-3- Ligand, thrombopoietin (TPO), Interleukin-6 (IL-6) for 24 hours at 37°C with one or more exogenous agents. Following cell treatments, levels of PD-L1 or IDO-1 cell surface protein were measured on the viable CD34+ cells by staining the cells with anti-CD34, anti-PD-L1 or anti-IDO-1, and 7- Aminoactinomycin D (7-AAD). Data was acquired on a FORTESSA® X-20 (Becton Dickinson) and analyzed using FLOWJO® (TreeStar).

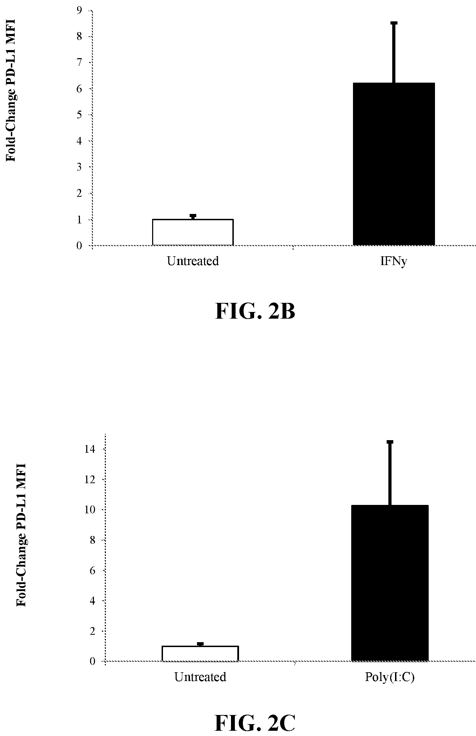

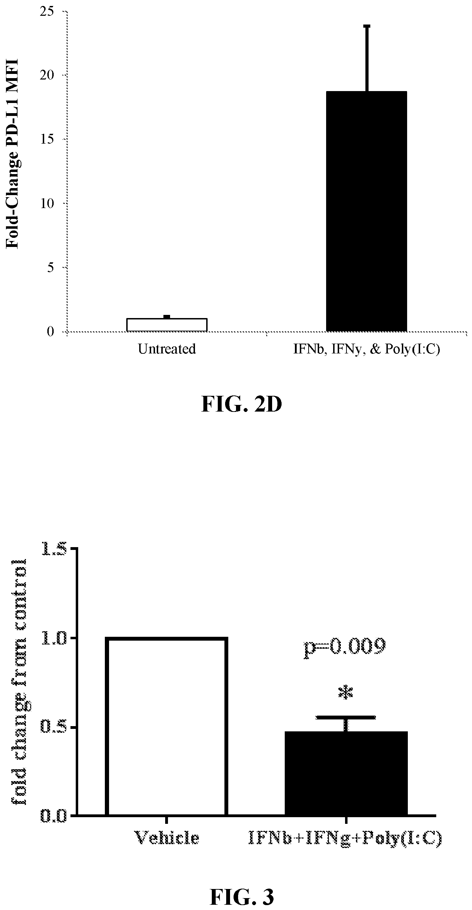

Fig. 2 shows the average fold-change of PD-L1 by median fluorescence intensity (MFI) relative to the untreated sample for three individual donors of CD34+ cells treated with a single exogenous agent (A) 1000U/mL IFNβ (B) 5ng/mL IFNγ (C) 10µg/mL Poly (I:C) or multiple exogenous agents D) 1000U/mL IFNβ, 5ng/mL IFNγ, and 10µg/mL Poly (I:C).

### EXAMPLE 3 - T cell proliferation is reduced in the presence of modulated HSPC.

HSCs were incubated 24 hours in media containing 1000 U/mL IFNβ, 5 ng/mL IFNγ, and 10 µg/mL Poly I:C or media containing vehicle. The cells were then washed and combined at a 1:1 ratio with autologous T cells. T cell mitogen was added and cocultures were incubated for 5 days. Fig. 3 shows T cell proliferation as measured by flow cytometry.

### EXAMPLE 4 - Time Course of PD-L1 Expression

Human CD34+ stem and progenitor cells (HSCs) isolated from mobilized peripheral blood were ex *vivo* treated in STEMSPAN® serum-free expansion medium (SFEM) (StemCell Technologies) with stem cell factor (SCF), Flt-3- Ligand, thrombopoietin (TPO), Interleukin-6 (IL-6) for 6, 24, or 48 hours at 37°C with one or more exogenous agents. Following cell treatments, levels of PD-L1 cell surface protein were measured on the viable CD34+ cells by staining the cells with anti-CD34, anti-PD-L1 and 7- Aminoactinomycin D (7-AAD). Data was acquired on a FORTESSA® X-20 (Becton Dickinson) and analyzed using FLOWJO® (TreeStar).

### Exogenous agents:

1000 U/mL IFNβ;
5 ng/mL IFNγ;
10 µg/mL Poly(I:C);
1000 U/mL IFNβ + 5 ng/mL IFNγ;
5 ng/mL IFNγ + 10 µg/mL Poly(I:C); or
1000 U/mL IFNβ + 5 ng/mL IFNγ+ 10 µg/mL Poly(I:C).

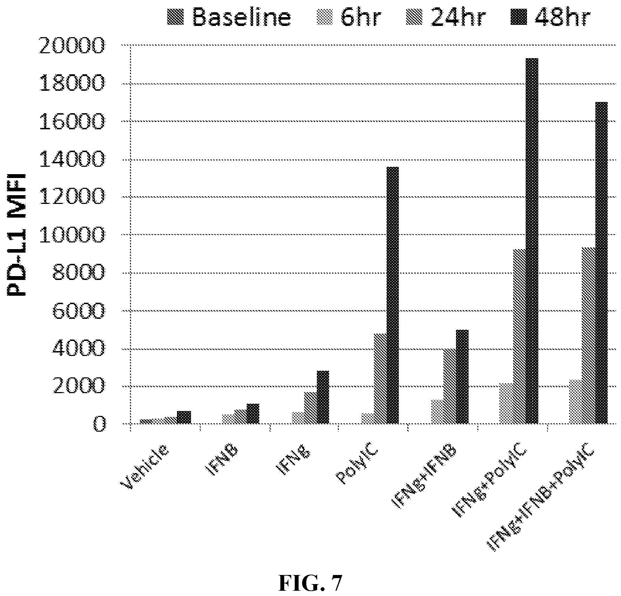

Fig. 7 shows the average fold-change of PD-L1 by median fluorescence intensity (MFI) relative to the untreated sample for three individual donors of CD34+ cells.

### EXAMPLE 5 - Post-Modulation Storage and PD-L1 Expression

Human CD34+ stem and progenitor cells (HSCs) isolated from mobilized peripheral blood were ex *vivo* treated in STEMSPAN® serum-free expansion medium (SFEM) (StemCell Technologies) with stem cell factor (SCF), Flt-3- Ligand, thrombopoietin (TPO), Interleukin-6 (IL-6) for 24 hours at 37°C with one or more exogenous agents. Following cell treatments, the media/reagents were washed, the cells were resuspended in either HBSS (for the "immediate" group), SFEM, or appropriate cryogenic media. Subsequently, the cells were either measured ("immediate") or placed for 24 hours at 37° C, 4°C, or were cryopreserved. After 24 hours, the cells were thawed or allowed to return to room temperature and levels of PD-L1 cell surface protein were measured on the viable CD34+ cells by staining the cells with anti-CD34 or anti-PD-L1, and 7- Aminoactinomycin D (7-AAD). Data was acquired on a FORTESSA® X-20 (Becton Dickinson) and analyzed using FLOWJO® (TreeStar).

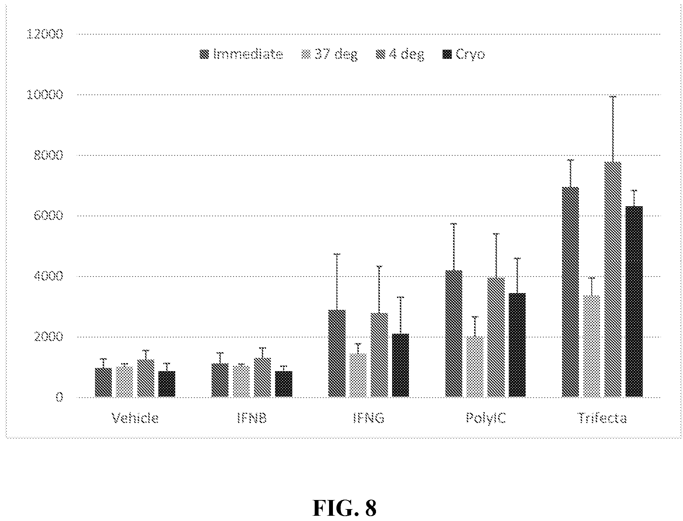

Fig. 8 demonstrates that post-modulation, the modulated cells are viable and express PD-L1 when maintained in a variety of conditions. Thus, the modulated cells may be stored post modulation.

### EXAMPLE 6 - Immunosuppression in Matched and Unmatched CD34+ Cell Populations

Human CD34+ stem and progenitor cells isolated from mobilized peripheral blood were ex vivo treated in StemSpan (StemCell Technologies) supplemented with stem cell factor (SCF), Flt-3-Ligand, thrombopoietin (TPO), Interleukin-6 (IL-6) and with IFNβ + IFNγ (Gamma+Beta) or IFNβ + IFNγ + Poly(I:C) (aka, the "Trifecta" group) for 6, 24, and 48 hours at 37°C. Following cell treatments, CD34 cells were washed and co-cultured with CD3/28 bead activated T cells. At the completion of a 5 day co-culture, the number of viable CD4 and CD8 T cells was quantified by staining the cells with anti-CD4, anti-CD8, and 7 Aminoactinomycin D (7-AAD). Data was acquired on a Fortessa X-20 (Becton Dickinson) and analyzed using FlowJo (TreeStar). The data is expressed as the fold change in the number of CD4+ and CD8+ T cells for each co-culture condition normalized to the number of T cells in the DMSO treated CD34 condition.

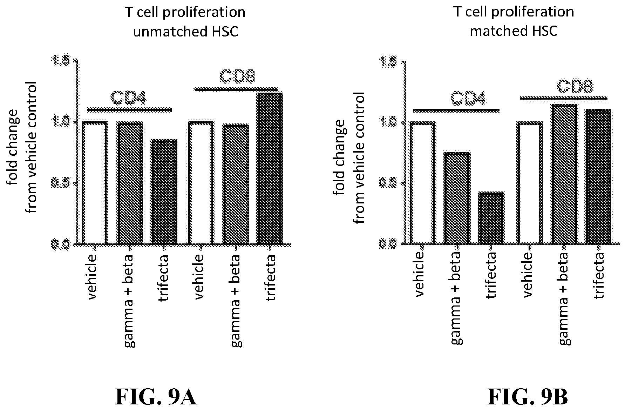

Figs. 9A and 9B Demonstrate that ex vivo treated human stem and progenitor cells suppress the proliferation of both autologous and allogeneic T cells.

### EXAMPLE 7 - Modulation of PD-L1 with PD-L1 Polynucleotides and Immunosuppression

Human CD34+ stem and progenitor cells isolated from mobilized peripheral blood were transduced with lentiviral vectors for the transgenic expression of human PD-L1 in StemSpan (StemCell Technologies) supplemented with stem cell factor (SCF), Flt-3-Ligand, thrombopoietin (TPO), Interleukin-6 (IL-6) for 24 hours at 37°C. Transduced CD34+ cells were isolated by flow cytometry using GFP reporter expression and the transduced (PD-L1⁺) or untransduced (PD-L1⁻) cells were co-cultured with CD3/28 bead activated T cells. At the completion of a 5 day co-culture, the number of viable CD4 and CD8 T cells was quantified by staining the cells with anti-CD4, anti-CD8, and 7 Aminoactinomycin D (7-AAD). Data was acquired on a Fortessa X-20 (Becton Dickinson) and analyzed using FlowJo (TreeStar). The data is expressed as the fold change in the number of CD4+ and CD8+ T cells for each co-culture condition normalized to the number of T cells in the DMSO treated CD34 condition.

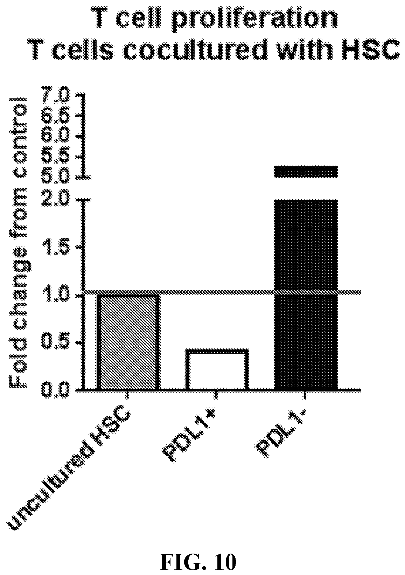

Fig. 10 demonstrates that genetic overexpression of PD-L1 in human CD34+ stem and progenitor cells enhances suppression of T cell proliferation.

### EXAMPLE 8 - Genetic overexpression of IDO-1 with IDO1 Polynucleotides and Immunosuppression

Human CD34+ stem and progenitor cells isolated from mobilized peripheral blood were transduced with lentiviral vectors for the transgenic expression of human IDO1 in StemSpan (StemCell Technologies) supplemented with stem cell factor (SCF), Flt-3-Ligand, thrombopoietin (TPO), Interleukin-6 (IL-6) for 24 hours at 37°C. Transduced CD34+ cells were isolated by flow cytometry using GFP reporter expression and the cells were co-cultured with CD3/28 bead activated autologous or allogeneic T cells in the presence or absence of the IDO1 inhibitor 1-methyl-D-tryptophan (1MT). At the completion of a 5 day co-culture, the number of viable CD4 and CD8 T cells was quantified by staining the cells with anti-CD4, anti-CD8, and 7 Aminoactinomycin D (7-AAD). Data was acquired on a Fortessa X-20 (Becton Dickinson) and analyzed using FlowJo (TreeStar). The data is expressed as the fold change in the number of CD4+ and CD8+ T cells for each co-culture condition normalized to the number of T cells in the untransduced CD34 condition.

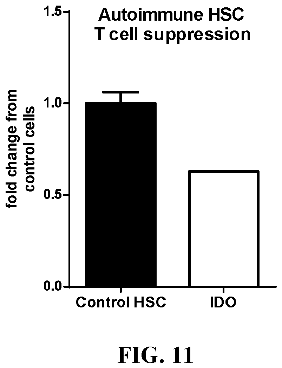

Fig. 11 demonstrates that genetic overexpression of IDO-1 in human CD34+ stem and progenitor cells enhances suppression of T cell proliferation.

### EXAMPLE 9 - Contact Independent Effect of Modulated Cells

Human CD34+ stem and progenitor cells isolated from mobilized peripheral blood were transduced with lentiviral vectors for the transgenic expression of human IDO-1 in StemSpan (StemCell Technologies) supplemented with stem cell factor (SCF), Flt-3-Ligand, thrombopoietin (TPO), Interleukin-6 (IL-6) for 24 hours at 37°C. Transduced CD34+ cells were isolated by flow cytometry using GFP reporter expression and the cells were co-cultured with CD3/28 bead activated autologous or allogeneic T cells in the presence or absence of the IDO-1 inhibitor 1-methyl-D-tryptophan (1MT). Media from transduced HSC were added to a population of human T-cells 1:1 by volume and incubated. At the completion of a 5 day incubation, the number of viable CD4 and CD8 T cells was quantified by staining the cells with anti-CD4, anti-CD8, and 7 Aminoactinomycin D (7-AAD). Data was acquired on a Fortessa X-20 (Becton Dickinson) and analyzed using FlowJo (TreeStar).

The data is expressed as the fold change in the number of CD4+ and CD8+ T cells for each co-culture condition normalized to the number of T cells in the untransduced CD34 condition.

### Results:

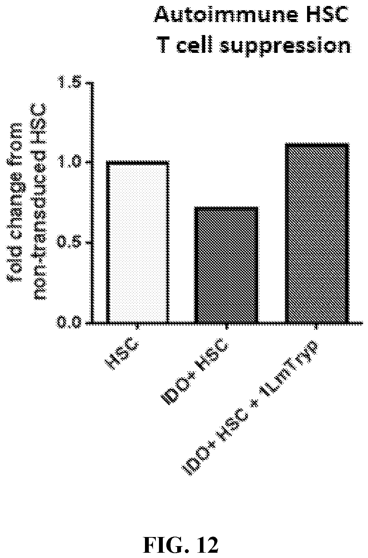

As shown by Fig. 12, T cells treated with media from a modulated population of hematopoietic cells demonstrating upregulated IDO-1 expression significantly suppressed T cell proliferation. The suppression of T cells demonstrates that modulated populations of cells suppress T cell responses in a contact-independent manner.

### EXAMPLE 10- Glucocorticoid Treated CD34+ Cells Decrease T Cell Proliferation and Effector Function in a Model of Type 1 Diabetes

### Methods

### Modification of Hematopoietic cells:

A population of CD34⁺ cells is contacted in HSC media with a number of different glucocorticoids for 24 hours at 37°C. Such contact significantly upregulates the expression of the T cell co-inhibitory protein, PDL-1. The PD-1/PDL-1 immune checkpoint pathway plays an important role in inhibiting the proliferation of autoreactive T cells, which in turn reduces autoimmunity and promotes self-tolerance. The population of CD34+ cells is washed and re-suspended in HBSS before injection to remove traces of the glucocorticoids and culture media.

### In Vivo Immunosuppression:

In order to determine the immune-suppressive potential of human CD34+ cells on diabetogenic T cells *in vivo,* 8 week old immuno-deficient NSG-HLA-A2/HHD mutant mice (which express the human HLA class 1 heavy and light chains) are injected intraperitoneally (i.p.) with 10x10⁶ HLA-A2+ peripheral blood mononuclear cells (PBMCs) from type 1 diabetic (T1D) patients. 3 days after the adoptive transfer of the T1D PBMCs, the animals are injected with either Hanks Balanced Salt Solution (HBSS), 10⁶ CD34⁺ cells isolated from mobilized peripheral blood of healthy patients grown 24 hours in hematopoietic stem cell (HSC) media (untreated-CD34), or 10⁶ CD34⁺ cells grown 24 hours in HSC media supplemented with a glucocorticoid (GC-CD34)(i.e., the modulated cells).

On days 7 and 14 post injection of the PBMCs, the spleen, bone marrow and pancreas are harvested. Human specific gene expression profiling, as well as flow cytometric analysis of the antibody stained cell preparation, is used to quantify the accumulation of human CD34⁺ and T cells in those tissues. Furthermore, the immuno-phenotype and effector function of the human T cells residing in the pancreas is assessed by flow cytometry, to characterize the differentiation and activation profiles of human T cells present in the target tissues.

Finally, using a separate cohort of mice, the pancreas is collected and prepared for immunofluorescence analysis of the Islets of Langerhans to determine β-cell insulin production and the nature of the inflammatory infiltrate. As above, human specific gene expression profiling and flow cytometric analysis is used to quantify the accumulation of human CD34⁺ and T cells in those tissues. Immuno-phenotype and effector function of the human T cells residing in the pancreas is assessed by flow cytometry, to characterize the differentiation and activation profiles of human T cells present in the target tissues.

### Results:

T cell proliferation and effector function is significantly reduced in the pancreas of the T1D PBMC animals injected with GC-CD34 cells both at day 7 and day 14 compared to vehicle-treated CD34s. This will be evidenced by a decrease in the proliferation of the human T cells and a reduction in their capacity to secrete INFγ after re-stimulation. In addition, as a result of the GC-CD34 mediated reduction of pancreatic T cell cytotoxicity, pancreatic β-cells have an increase in insulin production.

In summary, glucocorticoid treated CD34 cells are a more effective treatment of T cell mediated pancreatic β-cells toxicity compared to un-treated CD34 cells in this humanized T1D model.

The various embodiments described above can be combined to provide further embodiments. All of the U.S. patents, U.S. patent application publications, U.S. patent applications, foreign patents, foreign patent applications and non-patent publications referred to in this specification and/or listed in the Application Data Sheet are incorporated herein by reference, in their entirety. Aspects of the embodiments can be modified, if necessary to employ concepts of the various patents, applications and publications to provide yet further embodiments.
These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.
Also disclosed herein are *inter alia* the following items (said items are not claims):
1. A method for modulating a population of cells comprising:
   incubating the population of cells in the presence of one or more exogenous agents capable of increasing PD-L1 and/or IDO-1 expression to obtain a population of cells having increased expression of PD-L1 and/or IDO-1.
2. The method according to item 1, wherein the incubation is ex *vivo.*
3. The method according to item 1 or item 2, wherein the incubation is between about 5 minutes to about 72 hours.
4. The method according to any of items 1-3, wherein the incubation is between about 4 hours to about 48 hours.
5. The method according to any of items 1-4, wherein the incubation is performed at a temperature of between about 4°C to about 37°C.
6. The method according to any of items 1-5, wherein the incubation is performed at a temperature of about 37°C.
7. The method according to any of items 1-6, wherein the increase in PD-L1 and/or IDO-1 expression in the modulated cells is about 3-fold to about 80-fold compared to cells not incubated with the exogenous agent.
8. The method according to any of items 1-7, wherein the exogenous agent(s) are selected from polynucleotides, polypeptides, and small molecules.
9. The method according to any of item 8, wherein the small molecules comprise glucocorticoids, prostaglandin pathway agonists antineoplastics, dopamine receptor agonists, isometheptene mucate, dihydrostreptomycin sulfate, protriptyline, telenzepine, cyclobenzaprine, and 4-aminosalicylic acid.
10. The method according to item 8, wherein the polypeptide is an interferon receptor agonist selected from IFN-α, IFN-β, IFN-ε, IFN-κ, IFN-ω, IFN-γ, or a combination thereof.
11. The method according to any of items 1-10, wherein the population of cells is modulated with IFN-β and IFN-γ.
12. The method according to item 8, wherein the polynucleotide is selected from poly(I:C), a polynucleotide encoding PD-L1 and/or a polynucleotide encoding IDO-1.
13. The method according to item 9, wherein the prostaglandin pathway agonist is selected from PGE₂, dmPGE₂, 15(S)-15-methyl PGE₂, 20-ethyl PGE₂, 8-iso-16-cyclohexyl-tetranor PGE₂, 16,16-dimethyl PGE₂ ("dmPGE2"), p-(p-acetamidobenzamido) phenyl ester, 11-deoxy-16, 16-dimethyl PGE₂, 9-deoxy- 9-methylene-16, 16-dimethyl PGE₂, 9-deoxy-9-methylene PGE₂, 9-keto Fluprostenol, 5-trans PGE₂, 17-phenyl-omega-trinor PGE₂, PGE₂ serinol amide, PGE₂ methyl ester, 16-phenyl tetranor PGE₂, 15(S)-15-methyl PGE₂, 15(R)-15-methyl PGE₂, 8-iso-15-keto PGE₂, 8-iso PGE₂ isopropyl ester, 8-iso-16-cyclohexyl-tetranor PGE₂, 20-hydroxy PGE₂, 20-ethyl PGE₂, 11-deoxy PGE₁, nocloprost, sulprostone, butaprost, 15-keto PGE₂, and 19 (R) hydroxy PGE₂.
14. The method according to item 9, wherein the glucocorticoid is selected from medrysone, alclometasone, alclometasone dipropionate, amcinonide, beclometasone, beclomethasone dipropionate, betamethasone, betamethasone benzoate, betamethasone valerate, budesonide, ciclesonide, clobetasol, clobetasol butyrate, clobetasol propionate, clobetasone, clocortolone, cloprednol, cortisol, cortisone, cortivazol, deflazacort, desonide, desoximetasone, desoxycortone, desoxymethasone, dexamethasone, diflorasone, diflorasone diacetate, diflucortolone, diflucortolone valerate, difluorocortolone, difluprednate, fluclorolone, fluclorolone acetonide, fludroxycortide, flumetasone, flumethasone, flumethasone pivalate, flunisolide, flunisolide hemihydrate, fluocinolone, fluocinolone acetonide, fluocinonide, fluocortin, fluocoritin butyl, fluocortolone, fluorocortisone, fluorometholone, fluperolone, fluprednidene, fluprednidene acetate, fluprednisolone, fluticasone, fluticasone propionate, formocortal, halcinonide, halometasone, hydrocortisone, hydrocortisone acetate, hydrocortisone aceponate, hydrocortisone buteprate, hydrocortisone butyrate, loteprednol, meprednisone, 6a-methylprednisolone, methylprednisolone, methylprednisolone acetate, methylprednisolone aceponate, mometasone, mometasone furoate, mometasone furoate monohydrate, paramethasone, prednicarbate, prednisolone, prednisone, prednylidene, rimexolone, tixocortol, triamcinolone, triamcinolone acetonide and ulobetasol, as well as combinations thereof.
15. The method according to item 14, wherein the glucocorticoid is selected from betamethasone, clobetasol proprionate, flumethasone, flucinolone acetonide, medrysone, hydrocortisone, triamcinolone, alclometasone, and dexamethasone.
16. The method according to item 9, wherein the antineoplastics are selected from gemcitabine, letrozole, and fludarabine and the dopamine receptor antagonist is fluphernazine.
17. The method according to any of items 1-16, wherein the population of cells comprises hematopoietic cells.
18. The method according to any of items 1-17, wherein the population of cells is isolated.
19. The method according to any of items 1-18, wherein the population of hematopoietic cells is derived from cord blood, peripheral blood, bone marrow, or induced pluripotent stem cells (iPSCs).
20. The method according to any of items 1-19, wherein the population of hematopoietic cells is obtained from iPSCs.
21. The method according to any of items 1-20, wherein the population of cells comprises hematopoietic stem/progenitor cells (HSPCs).
22. The method according to any of items 1-21, wherein the population of cells comprises at least about 50%, at least about 60%, at least about 70%, at least about 80%, at least about 85%, at least about 90%, at least about 95%, at least about 98%, or at least about 99% HSPCs.
23. The method according to item 22, wherein the population of cells comprises a substantially pure population of HSPCs.
24. The method according to any of items 1-23, wherein the population of cells is enriched for CD34+ HPSCs prior to contact with the exogenous agent.
25. A population of cells having increased PD-L1 and/or IDO-1 expression obtained by the method according to any of items 1-24.
26. A method of treating an immunological disorder comprising administering a therapeutically effective amount of the population of cells according item 25 to a patient in need thereof.
27. The method according to item 26, wherein the population of cells comprises hematopoietic cells.
28. The method according to item 26 or item 27, wherein the population of hematopoietic cells is derived from cord blood, peripheral blood, bone marrow, or iPSCs.
29. The method according to any of items 26-28, wherein the population of cells comprises HSPCs.
30. The method according to any of items 26-29, wherein the population of cells comprises at least about 50%, at least about 50%, at least about 60%, at least about 70%, at least about 80%, at least about 85%, at least about 90%, at least about 95%, at least about 98%, or at least about 99% HSPCs.
31. The method according to item 30, wherein the population of cells comprises a substantially pure population of HSPCs.
32. The method according to any of items 26-31, wherein the population of cells is enriched for CD34+ HPSCs prior to contact with the exogenous agent.
33. The method according to any of items 26-32, wherein the population of cells is allogeneic to the patient.
34. The method according to any of items 26-33, wherein the population of cells is HLA matched with the patient.
35. The method according to any of items 26-34, wherein the population of cells comprises haplotyped enhanced-HSPCs.
36. The method according to any of items 26-35, wherein the population of cells is partially HLA matched or unmatched with the patient.
37. The method according to any of items 26-36, wherein the therapeutically effective amount of the population of cells comprises about 2 x 10⁶ to about 2 x 10¹⁰ CD34+ hematopoietic cells.
38. The method according to any of items 26-37, wherein the method comprises more than one administration of a therapeutically effective amount of cells.
39. The method according to any of items 26-38, wherein the frequency of administrations ranges from about every other week to about every six months.
40. The method according to any of items 26-39, wherein the initial administration is a higher number of cells than a subsequent administration.
41. The method according to any of items 26-40, wherein the immunological disorder is an autoimmune disorder selected from acute myocardial infarction, ischemic stroke, type 1 diabetes, diabetes mellitus, multiple sclerosis, acute disseminated encephalomyelitis, inflammatory demyelinating diseases, lupus, Crohn's disease, osteoarthritis, rheumatoid arthritis, psoriatic arthritis, ulcerative colitis, dermatitis, irritable bowel syndrome, vitiligo, Graves' disease, Hashimoto's disease, Addison's disease, polymyositis, dermatomyositis, myasthenia gravis, autoimmune hepatitis, Sjögren's syndrome, autoimmune gastritis, sclerosis, psoriasis, asthma, or Wegener's granulomatosis.
42. The method according to any of items 26-41, wherein the immunological disorder is graft vs host disease or transplant rejection.
43. The method according to item 42, wherein the transplant rejections arose from a bone marrow transplant, solid organ transplant, or cell therapy (e.g. any composition comprising isolated stem cells).
44. The method according to any of items 26-43, wherein the patient has not undergone conditioning.
45. The method according to any of items 26-43, wherein the patient has not undergone at least one of high-dose, reduced-intensity, or nonmyeloablative conditioning.
46. The method according to any of items 26-43, wherein the patient has undergone at least one of high-dose, reduced-intensity, or nonmyeloablative conditioning.
47. The method according to any of items 26-46, wherein the patient is not a candidate for cellular transplant or has not received a transplant.
48. A method of treating inflammation in a patient comprising:
   administering a therapeutically effective amount of a composition comprising a population of cells according to item 25 to a patient in need thereof.
49. The method according to item 48, wherein the population of cells comprises hematopoietic cells.
50. The method according to item 48 or item 49, wherein the population of hematopoietic cells is derived from cord blood, peripheral blood, bone marrow, or iPSCs.
51. The method according to any of items 48-50, wherein the population of cells comprises HSPCs.
52. The method according to any of items 48-51, wherein the population of cells comprises at least about 50% HPSCs, at least about 50%, at least about 60%, at least about 70%, at least about 80%, at least about 85%, at least about 90%, at least about 95%, at least about 98%, or at least about 99% HSPCs.
53. The method according to item 52, wherein the population of cells comprises a substantially pure population of HSPCs.
54. The method according to any of items 48-53, wherein the population of cells is enriched for CD34+ HPSCs prior to contact with the exogenous agent.
55. The method according to any of items 48-54, wherein the population of cells is allogeneic to the patient.
56. The method according to any of items 48-55, wherein the population of cells is HLA matched with the patient.
57. The method according to any of items 48-56, wherein the population of cells comprises haplotyped enhanced-HSPCs.
58. The method according to any of items 48-57, wherein the population of cells is partially HLA matched or unmatched with the patient.
59. The method according to any of items 48-58, wherein the therapeutically effective amount of the population of cells comprises about 2 x 10⁶ cells to about 2 x 10¹⁰ CD34+ hematopoietic cells.
60. The method according to any of items 48-59, wherein the method comprises more than one administration of a therapeutically effective amount of cells.
61. The method according to any of items 48-60, wherein the frequency of administrations ranges from about every other week to about every six months.
62. The method according to any of items 48-61, wherein the initial administration is a higher number of cells than a subsequent administration.
63. The method according to any of items 48-62, wherein the inflammatory disorder is selected from inflammation of the lungs, joints, connective tissue, eyes, nose, bowel, kidney, liver, skin, central nervous system, endocrine system, cardiovascular system and heart.
64. The method according to item 63, wherein the inflammation of the lung is selected from asthma, adult respiratory distress syndrome, bronchitis, pulmonary inflammation, pulmonary fibrosis, and cystic fibrosis.
65. The method according to item 63, wherein the inflammation of the joints is selected from rheumatoid arthritis, rheumatoid spondylitis, juvenile rheumatoid arthritis, osteoarthritis, gouty arthritis and other arthritic conditions.
66. The method according to item 63, wherein the inflammation of the eye is selected from uveitis (including iritis), conjunctivitis, scleritis, keratoconjunctivitis sicca, and retinal diseases, including, but not limited to, diabetic retinopathy, retinopathy of prematurity, retinitis pigmentosa, and dry and wet age-related macular degeneration.
67. The method according to item 63, wherein the inflammation of the bowels is selected from Crohn's disease, ulcerative colitis and distal proctitis.
68. The method according to item 63, wherein the inflammation of the skin is selected from psoriasis, eczema and dermatitis, (e.g., eczematous dermatitides, topic and seborrheic dermatitis, allergic or irritant contact dermatitis, eczema craquelee, photoallergic dermatitis, phototoxic dermatitis, phytophotodermatitis, radiation dermatitis, and stasis dermatitis), scleroderma, ulcers and erosions resulting from trauma, burns, bullous disorders, or ischemia of the skin or mucous membranes, several forms of ichthyoses, epidermolysis bullosae, hypertrophic scars, keloids, cutaneous changes of intrinsic aging, photoaging, frictional blistering caused by mechanical shearing of the skin, cutaneous atrophy resulting from the topical use of corticosteroids, cheilitis, chapped lips, nasal irritation, mucositis and vulvovaginitis.
69. The method according to item 63, wherein the inflammation of the endocrine system is selected from autoimmune thyroiditis (Hashimoto's disease), Type I diabetes, Type II diabetes, and acute and chronic inflammation of the adrenal cortex.
70. The method according to item 63, wherein the inflammation of the cardiovascular system are selected from coronary infarct damage, peripheral vascular disease, myocarditis, vasculitis, revascularization of stenosis, artherosclerosis, and vascular disease associated with Type II diabetes.
71. The method according to item 63, wherein the inflammation of the kidney is selected from glomerulonephritis, interstitial nephritis, lupus nephritis, nephritis secondary to Wegener's disease, acute renal failure secondary to acute nephritis, Goodpasture's syndrome, post-obstructive syndrome and tubular ischemia.
72. The method according to item 63, wherein the inflammation of the liver is selected from hepatitis (arising from viral infection, autoimmune responses, drug treatments, toxins, environmental agents, or as a secondary consequence of a primary disorder), biliary atresia, primary biliary cirrhosis and primary sclerosing cholangitis.
73. The method according to item 63, wherein the inflammation of the central nervous system is selected from multiple sclerosis and neurodegenerative diseases such as Alzheimer's disease, Parkinson's disease, or dementia associated with HIV infection.
74. The method according to any of items 48-73, wherein the administration is systemic.
75. The method according to any of items 48-73, wherein the administration is local.
76. The method according to any of items 48-75, wherein the administration is intravenous, intraarterial, intramuscular, intrathecal, intracapsular, intraorbital, intracardiac, intradermal, intraperitoneal, transtracheal, subcutaneous (subdermal), subcuticular, intraarticular, subcapsular, subarachnoid, intraspinal, intrastemal injection, or by infusion.
77. The method according to any of items 48-76, wherein the method is part of a combination therapy.
78. A pharmaceutical composition comprising a population of modulated hematopoietic cells that expresses an increased level of PD-L1 and/or IDO-1 expression that is about 3 fold to about 80 fold compared to a level of PD-L1 and/or IDO-1 expression in a population of non-modulated hematopoietic cells.
79. The pharmaceutical composition according to item 78, wherein the pharmaceutical composition further comprises a pharmaceutically acceptable carrier.
80. The pharmaceutical composition according to item 78 or item 79, wherein the population of hematopoietic cells is derived from cord blood, peripheral blood, bone marrow, or iPSCs.
81. The pharmaceutical composition according to any of items 78-80, wherein the population of hematopoietic cells is derived from differentiated iPSCs.
82. The pharmaceutical composition according to any of items 78-81, wherein the population of hematopoietic cells comprises HSPCs.
83. The pharmaceutical composition according to any of items 78-82, wherein the population of hematopoietic cells comprises at least about 50% HPSCs, at least about 50%, at least about 60%, at least about 70%, at least about 80%, at least about 85%, at least about 90%, at least about 95%, at least about 98%, or at least about 99% HSPCs.
84. The method according to item 83, wherein the population of hematopoietic cells comprises a substantially pure population of HSPCs.
85. The pharmaceutical composition according to any of items 78-84, wherein the population of hematopoietic cells is enriched for CD34+ HPSCs.
86. The pharmaceutical composition according to any of items 78-85, wherein the composition is formulated for intravenous administration, intraarterial administration, intramuscular administration, intrathecal administration, intracapsular administration, intraorbital administration, intracardiac administration, intradermal administration, intraperitoneal administration, transtracheal administration, subcutaneous (subdermal) administration, subcuticular administration, intraarticular administration, subcapsular administration, subarachnoid administration, intraspinal administration, intrastemal administration, and infusion.
87. The pharmaceutical composition according to any of items 78-86, wherein the pharmaceutical composition is formulated for a local or non-intravenous administration.
88. The pharmaceutical composition according to any of items 78-87, wherein the population of hematopoietic cells comprises about 2 x 10⁶ to about 2 x 10¹⁰ CD34+ hematopoietic cells.
89. A kit comprising the pharmaceutical composition according to any of items 78-89 and a second active agent for use in a combination therapy.

## Claims

1. A population of modified hematopoietic stem cells (HSCs), wherein the HSCs have been modified to comprise an exogenous copy of a polynucleotide encoding PD-L1.

2. A pharmaceutical composition comprising: (a) the population of modified HSCs according to claim 1, and (b) a pharmaceutically acceptable carrier.

3. A method of treating an immunological disorder, comprising the step of: administering to a patient with an immunological disorder the pharmaceutical composition of claim 2, wherein the population of modified HSCs express PD-L1 at a higher level than a population of unmodified HSCs.
